(19) Europäisches Patentamt
European Patent Office
Office européen des brevets

(11) **EP 2 346 407 B1**

(12) **EUROPEAN PATENT SPECIFICATION**

(45) Date of publication and mention
of the grant of the patent:
**28.11.2018 Bulletin 2018/48**

(21) Application number: **09814316.7**

(22) Date of filing: **17.09.2009**

(51) Int Cl.:
*A61B 6/03* *(2006.01)*        *A61B 5/00* *(2006.01)*
*A61B 10/00* *(2006.01)*       *G01T 1/161* *(2006.01)*

(86) International application number:
**PCT/JP2009/004692**

(87) International publication number:
**WO 2010/032471 (25.03.2010 Gazette 2010/12)**

(54) **A DEVICE FOR GENERATING ALTERNATIVE OF NORMAL BRAIN DATABASE**

VORRICHTUNG ZUR ERZEUGUNG EINER ALTERNATIVE ZU EINER NORMALEN
GEHIRNDATENBANK

DISPOSITIF DE GÉNÉRATION D'UNE BASE DE DONNÉES ALTERNATIVE POUR CERVEAU
NORMAL

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **22.09.2008 US 234910**

(43) Date of publication of application:
**27.07.2011 Bulletin 2011/30**

(73) Proprietors:
• **University of Washington**
  **Seattle, WA 98105-4608 (US)**
• **Nihon Medi-Physics Co., Ltd.**
  **Tokyo 136-0075 (JP)**

(72) Inventors:
• **MINOSHIMA, Satoshi**
  **Seattle, Washington 9819-57115 (US)**
• **WATANABE, Kiyotaka**
  **Tokyo 136-0075 (JP)**
• **MIKI, Shuya**
  **Tokyo 136-0075 (JP)**
• **NISHIKAWA, Kazuhiro**
  **Nishinomiya-shi**
  **Hyogo 662-0918 (JP)**

(74) Representative: **Ström & Gulliksson AB**
  **P O Box 4188**
  **203 13 Malmö (SE)**

(56) References cited:
**WO-A1-2008/093057      US-A1- 2007 081 699**

• **YAMAMOTO YASUSHI ET AL.: 'The evaluation of
Cerebral Blood Flow SPECT by Statistics Image
Analysis (Report from the Scientific Research
Group)' JAPANESE JOURNAL OF
RADIOLOGICAL TECHNOLOGY vol. 64, no. 6, 20
June 2008, pages 752 - 765, XP008137096**
• **MATSUO TAICHI ET AL.: '253 Study on
Generating Normal Database for Cerebral Blood
Flow SPECT by Statistics Image Analysis (253
nouketuryu SPECT toukeigakuteki kaisekihou ni
okeru seijyo detabeisu sakusei ni tsuiteno kento)'
JAPANESE JOURNAL OF RADIOLOGICAL
TECHNOLOGY vol. 63, no. 9, 20 September 2007,
page 1049, XP008138422**

**Description**

**[Technical Field]**

The present invention relates to the generation of an alternative of a normal brain database (hereinafter referred as an "alternative of a normal brain database" which can be used for diagnosing diseases based on functional images of the brain such as PET (Positron Emission Tomography) images and SPECT (Single Photon Emission Computed Tomography) images.

**[Background Art]**

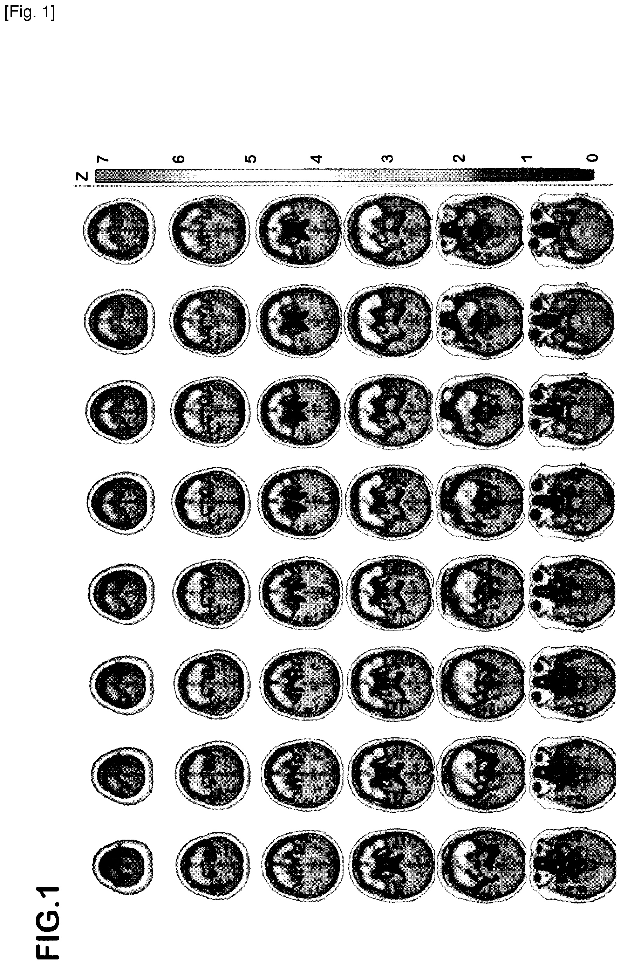

To diagnose Alzheimer's disease etc., measuring and imaging functional status such as bloodstream or glucose metabolism of each point of the patient's brain is carried out. In Positron Emission Tomography (hereinafter referred as "PET"), medical agents whichare indicated by nuclear positron emission, such as $^{18}$F-FDG, are injected to obtain the functional status such as the glucose metabolism by measuring the gamma ray amount as annihilation radiation at each point of the patient's brain. In Single Photon Emission Computed Tomography (hereinafter referred as "SPECT"), gamma ray emission nuclear species such as $^{123}$I and $^{99m}$Tc are used for the same purpose. As shown in Fig.1, a plurality of sectional images are generated by measuring the gamma ray amount of each section of the patient's brain. In the sectional image, for example, red, yellow, green and blue are used for showing areas in descending order of status value such as the bloodstream or the glucose metabolism associated value (voxel value associating functional value measured by PET of SPECT).

Diagnosis of disease can be carried out by comparing data showing the status value of normal healthy subjects and data showing the status value of the patient. For comparison, a computer displays a differential image between the sectional image of the normal healthy subject and the sectional image of the patient. To obtain the differential image, the sectional image of the patient is spatially fitted to that of a normal healthy subject and a Z-score at each point is calculated. Methods to achieve such diagnosis is well known, such as 3-Dimentional Stereotaxic Surface Projection (3D-SSP) developed by Minoshima of Washington University, and Statistical Parametoric Mapping (SPM) developed by Friston et al. of Hammersmith Hospital, U.K.

The data showing the status value of a normal healthy subject comprises the mean and standard deviation of the status values of each point, which are obtained from a plurality of normal healthy subjects. The data showing the status value of a normal healthy subject is called a normal brain database. The Z-score is obtained by dividing the difference between the status value of the patient and the status value of the normal healthy subject at each point by the standard deviation at each point of a normal brain database. See equation (1).

$$Z(x,y,z) = (I_{mean}(x,y,z) - I(x,y,z)) / SD(x,y,z) \ (1)$$

where $Z(x,y,z)$ is the Z-score at the point of the coordinate x,y,z, $I_{mean}(x,y,z)$ is the mean value of the status values (voxel values associated to functional status measured by PET of SPECT etc.) at said point of the normal healthy subjects, $I(x,y,z)$ is the status value at said point of the patient and $SD(x,y,z)$ is the standard deviation of status values at the point of normal healthy subjects. $I_{mean}(x,y,z)$ and $SD(x,y,z)$ can be obtained from the normal brain database.

In this method, the difference between a normal healthy subject and a patient can be clearly shown by using the normal brain database with standard deviation.

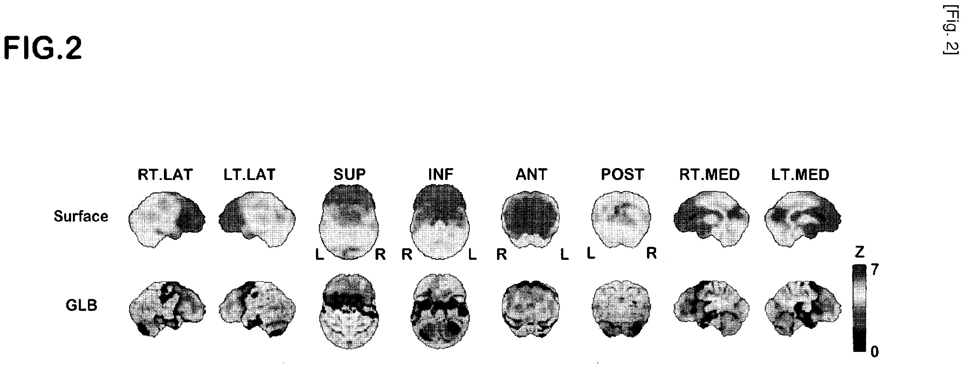

In 3D-SSP, the biggest status value from the brain surface to a predetermined depth perpendicular to the brain surface, is selected as a representative status value and is displayed on the brain surface. Then, a Z-score is calculated by comparing the selected status values of a patient with that of normal healthy subjects. Images of the Z-score are displayed as right-brain lateral surface RT-LAT, left-brain lateral surface LT-LAT, top surface SUP, bottom surface INF, anterior surface ANT, posterior surface POST, right-brain medial surface R-MED and left-brain medial surface L-MED as shown in Fig. 2. In Fig. 2, upper images (denoted "surface") show status values of brain surface and lower images (denoted "GLB") show the Z-score of brain surface. The Z-score image enables the improvement of the detection ability of diseases and to assess the severity of diseases.

Mean values and standard deviations of selected status values of the brain surface (said selected biggest values) at each point of the brain surface of a plurality of normal healthy subjects should be provided as a normal brain database in the 3D-SSP method.

To achieve a high diagnosing ability, the normal brain database is made based on preferably at least 10 normal healthy subjects. See Chen WP et al., "Effect of sample size for normal brain database on diagnostic performance of brain FDG PET for the detection of Alzheimer's disease using automated image analysis" Nucl Med Commun. 2008 Mar;29(3):270-6.

It is not easy to gather image data of normal healthy subjects, because most of functional brain images such as PET images and SPECT images gathered by the medical center are the functional brain images of subjects who visit the medical center and who possibly have a disease. Further, functional brain images may vary according to radio isotopes corresponding to the disease to be diagnosed and materials indicating them. Therefore, a normal brain database should be generated for each combination of radio isotopes and materials indicating them. The above mentioned situations disturb the generation of a normal brain database.

**[Citation List]**

**[Non Patent Literature]**

[NPL 1]
Chen WP et al., "Effect of sample size for normal brain database on diagnostic performance of brain FDG PET for the detection of Alzheimer's disease using automated image analysis" Nucl Med Commun. 2008 Mar;29(3):270-6

**[SummaryofInvention]**

**[Technical Problem]**

In the following description, a "brain status image" means an image which shows the status of a brain such as a brain functional image.
It is an object of the present invention to provide a device and a method which can easily generate an alternative of a normal brain database which can be used as an alternative for the normal brain database which is difficult to generate.

**[Solution to Problem]**

(1) A device according to claim 1.
(2) In one embodiment of the present invention, the rejecting means rejects the extreme deviate brain status values, statistically obtained at each point, when said extreme deviate brain status values are presumed to indicate a disease.
(3) In one embodiment of the present invention, the brain status image is a brain bloodstream image which shows the bloodstream associated value (voxel value obtained by PET or SPECT etc. associated with the bloodstream value) of each point of the brain.
(4)
In one embodiment of the present invention, the number m of rejecting the smallest value to the smallest m-th is larger than the number n of rejecting the largest value to the largest n-th.
(5) In one embodiment of the present invention, the generating means generates the alternative of a normal brain database by obtaining mean a status value and a standard deviation for each said point of brain to be used for diagnosing disease based on said anatomically transformed and normalized brain status images of subjects in which said status values presumed to indicate disease are rejected.
(6) A device for generating an alternative of a normal brain database wherein the embodying of the present invention further comprises:

means for generating a brain surface status image of each subject based on said anatomically transformed and normalized brain status images of the subjects, said brain surface status images having surface status value of each surface portion, said surface status value being selected as a representative value of status values from the brain surface to a predetermined depth perpendicular to the brain surface; and
wherein said each surface point at which said surface status value is indicated is used as said point of brain to be used for diagnosing disease.

A brain surface bloodstream image or a brain surface glucose metabolism image as the brain surface status image, can be generated by bloodstream values of a brain bloodstream image or glucose metabolisms of a brain glucose metabolism image, respectively.
(7) A computer program according to claim 7-12.

A method according to claim 13-18.

The forgoing forms and other forms, objects, and aspects as well as features and advantages of the present invention will become further apparent from the following detailed description of the presently preferred embodiments, read in conjunction with the accompanying drawings. The detailed description and drawings are merely illustrative of the present invention rather than limiting the scope of the present invention being defined by the appended claims.

[Brief Description of Drawings]

[Fig. 1]
Fig. 1 shows a plurality of sectional images of the brain bloodstream associated value.
[Fig. 2]
Fig. 2 shows images of the brain surface bloodstream associated value.
[Fig. 3]

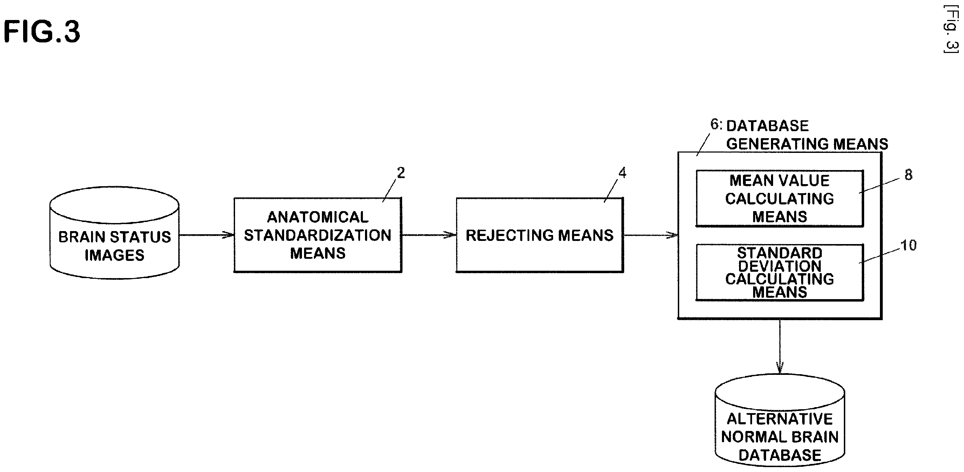

Fig. 3 shows a block diagram of a device for generating an alternative to a normal brain database (hereinafter referred to as "an alternative of a normal brain database").
[Fig. 4]

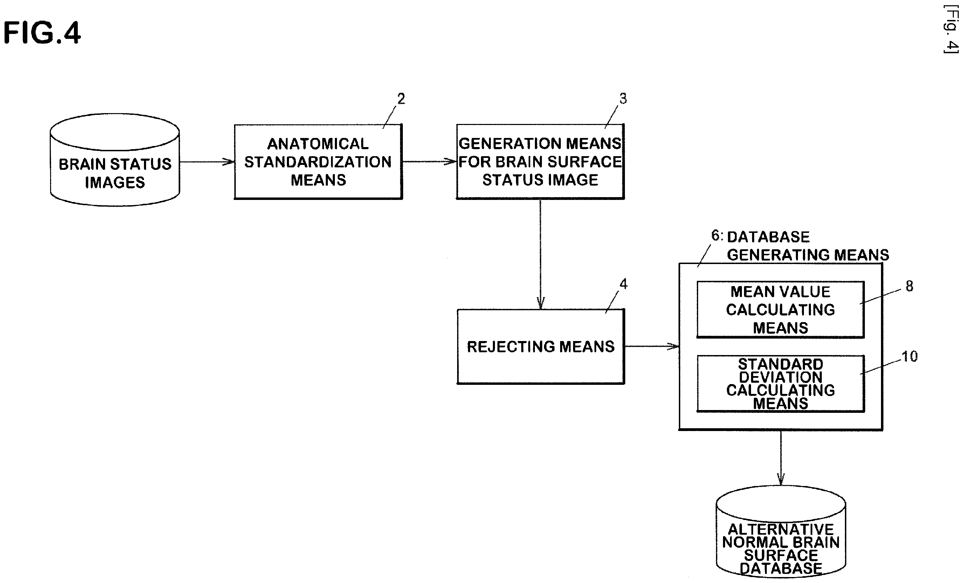

Fig. 4 shows a block diagram of a device for generating an alternative of a normal brain database in another embodiment of the present invention.
[Fig. 5]

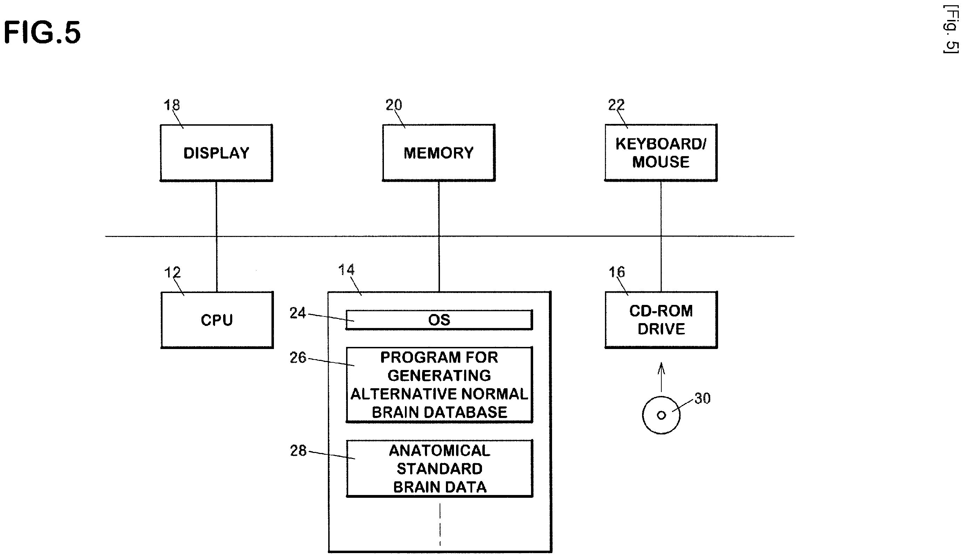

Fig. 5 shows a hardware construction of the device for generating an alternative of a normal brain database.
[Fig. 6]

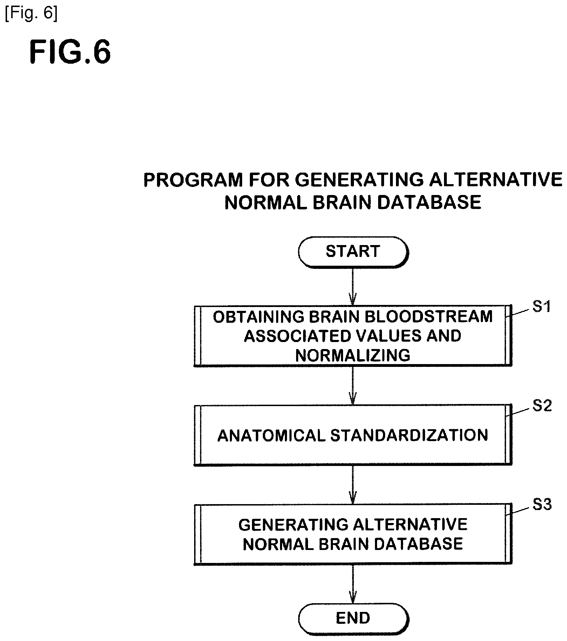

Fig. 6 is a flowchart of the program for generating an alternative of a normal brain database stored in a hard-drive.
[Fig. 7]

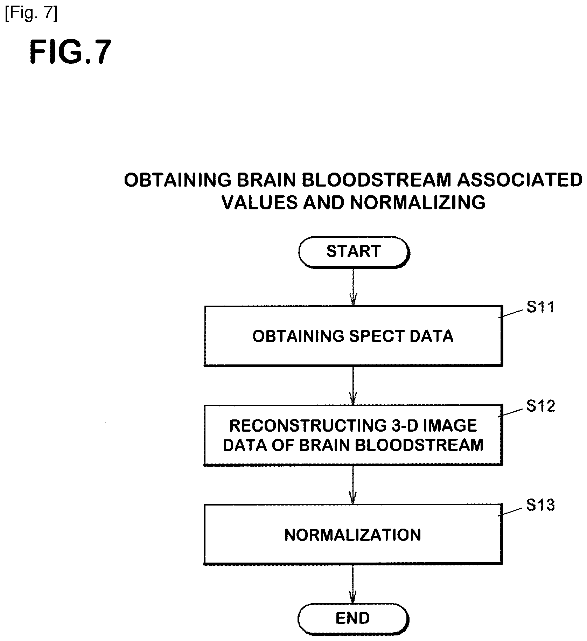

Fig. 7 is a detailed flowchart for obtaining brain bloodstream data (step S1 of Fig.6)
[Fig. 8]

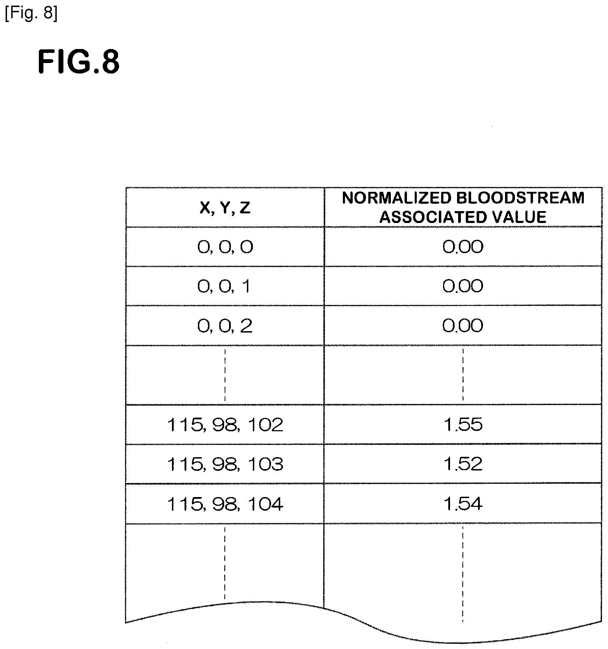

Fig. 8 shows a table of normalized brain bloodstream data.
[Fig. 9]

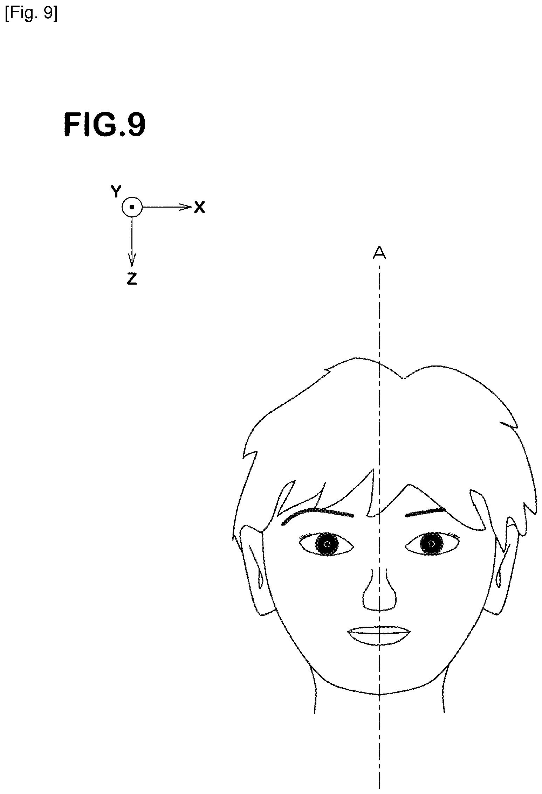

Fig. 9 shows the X axis, Y axis and Z-axis relating to the cephalon.
[Fig. 10]

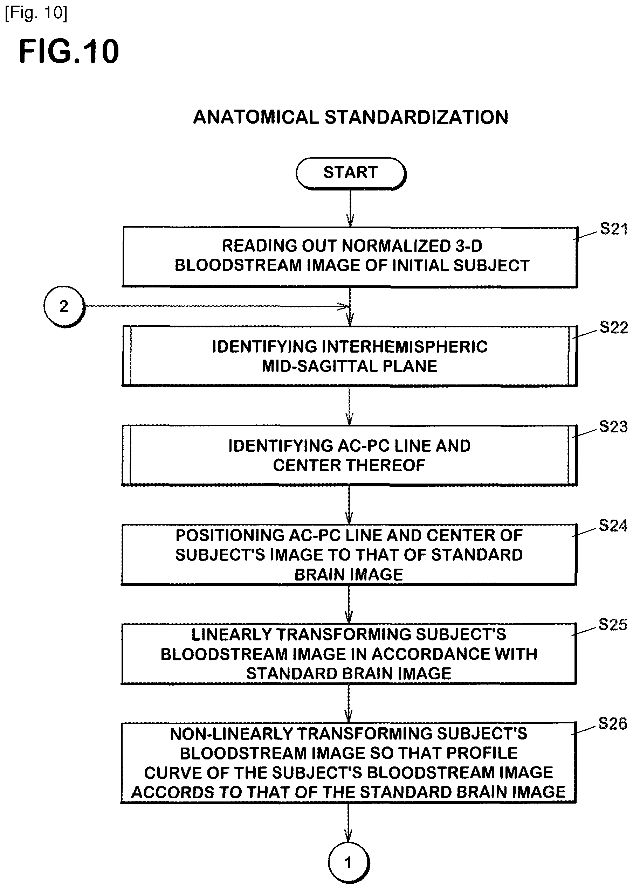

Fig. 10 shows a detailed flowchart for standardizing the bloodstream image (step S2 of Fig.6).
[Fig. 11]

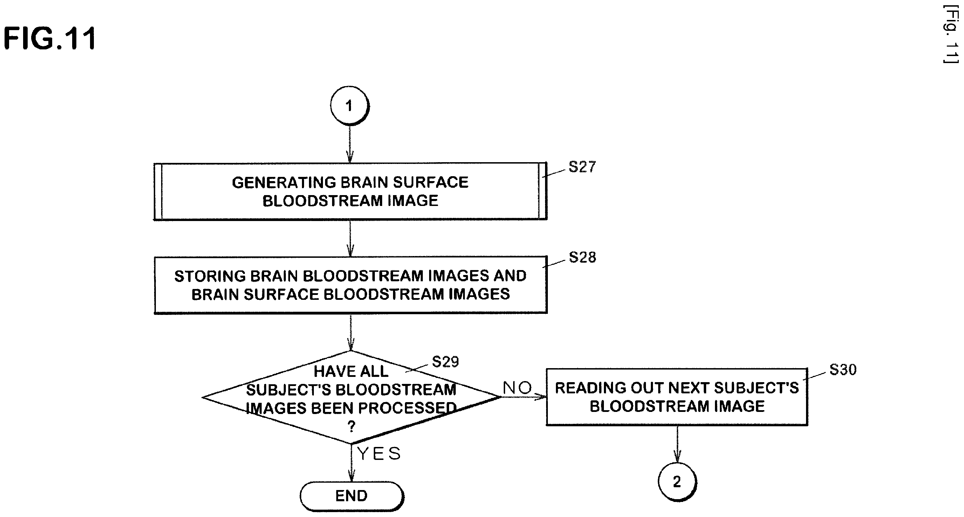

Fig. 11 shows a detailed flowchart for standardizing the bloodstream image (step S2 of Fig.6).
[Fig. 12]

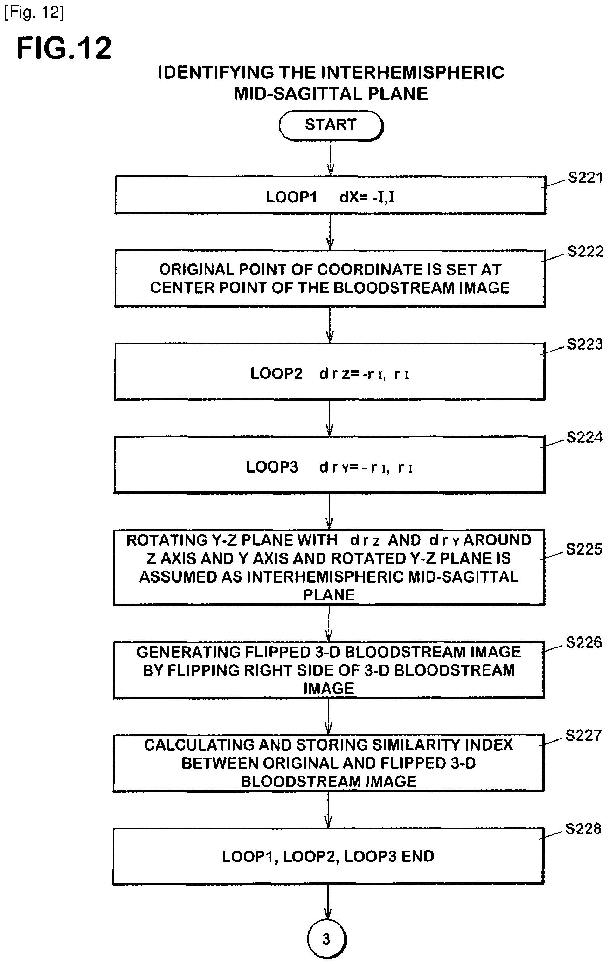

Fig. 12 shows a detailed flowchart for identifying the interhemispheric mid-sagittal plane of the bloodstream image (step S22 of Fig.10).
[Fig. 13]

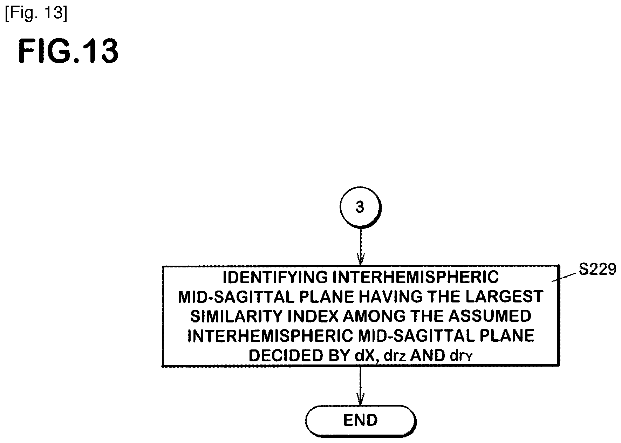

Fig. 13 shows a detailed flowchart for identifying the interhemispheric mid-sagittal plane of the bloodstream image (step S22 of Fig.10).
[Fig. 14]

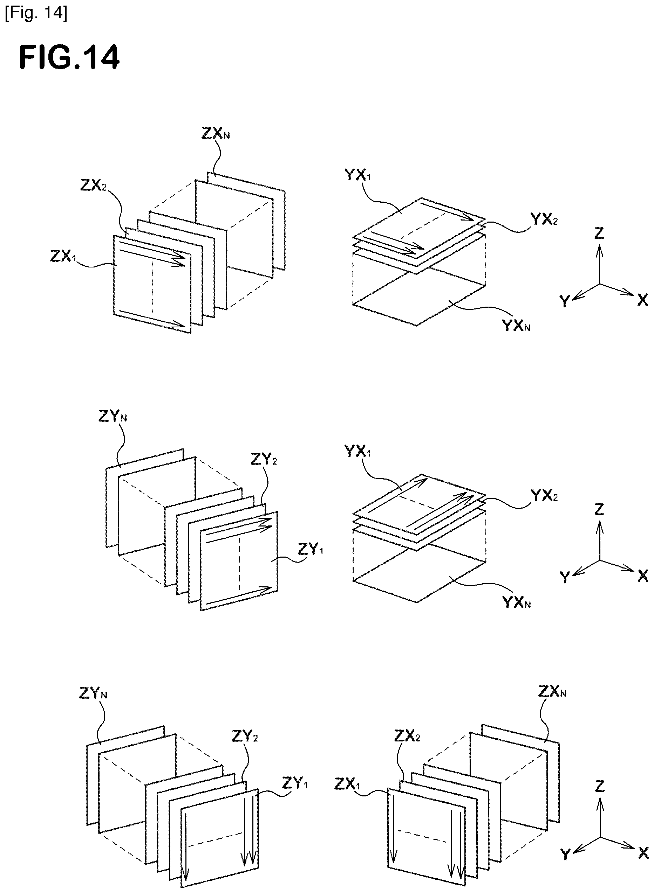

Fig. 14 shows a calculation of the stochastic sign change (SSC).
[Fig. 15]

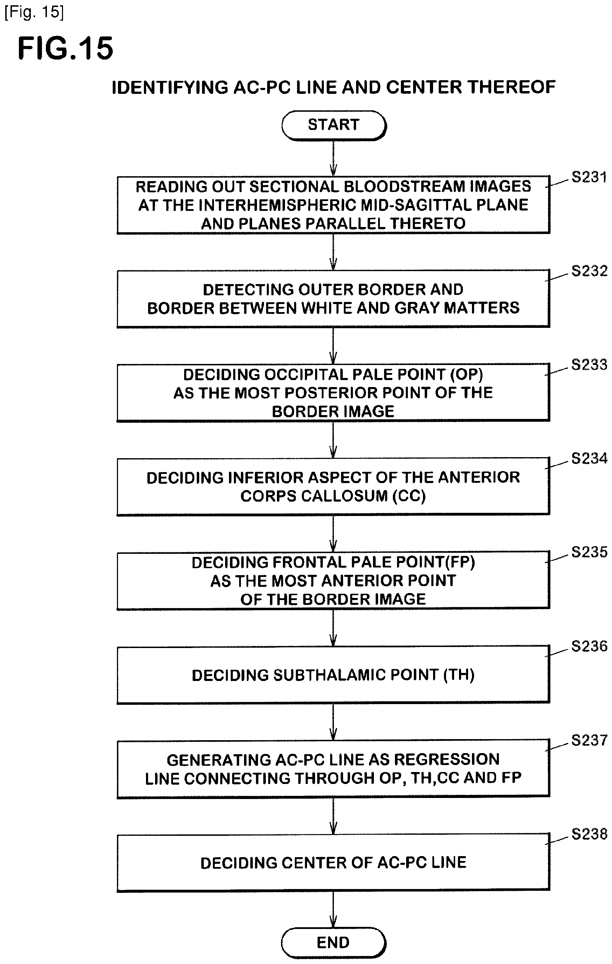

Fig. 15 is a detailed flowchart for identifying the AC-PC line and the center thereof (step S23 of Fig.10).
[Fig. 16]

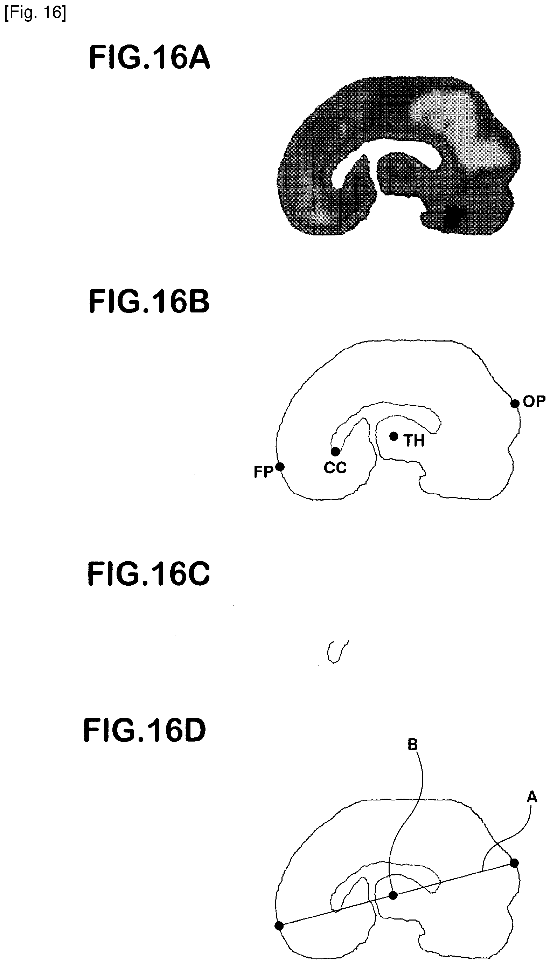

Fig. 16 shows steps for identifying the AC-PC line.
[Fig. 17]

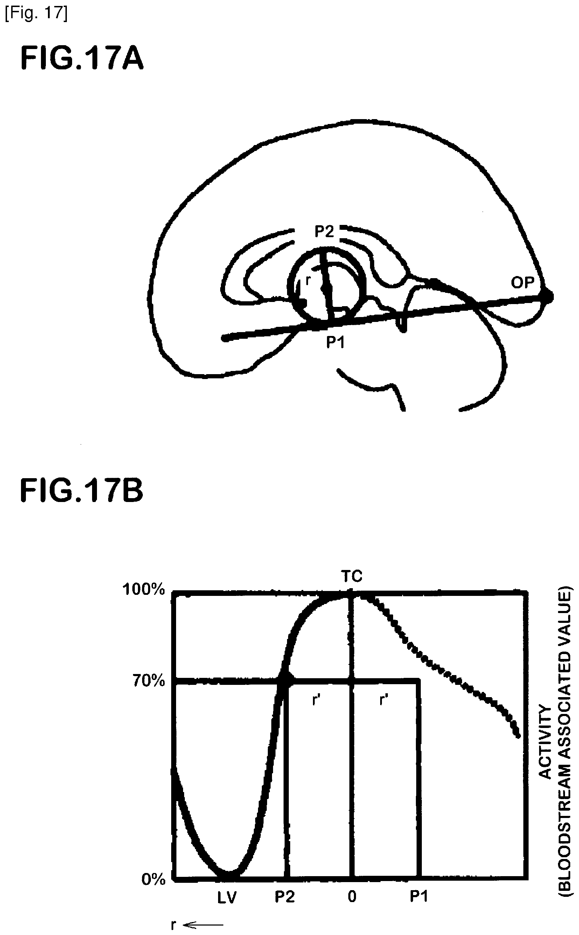

Fig. 17 shows an estimation of the landmark TH for identifying the AC-PC line.
[Fig. 18]

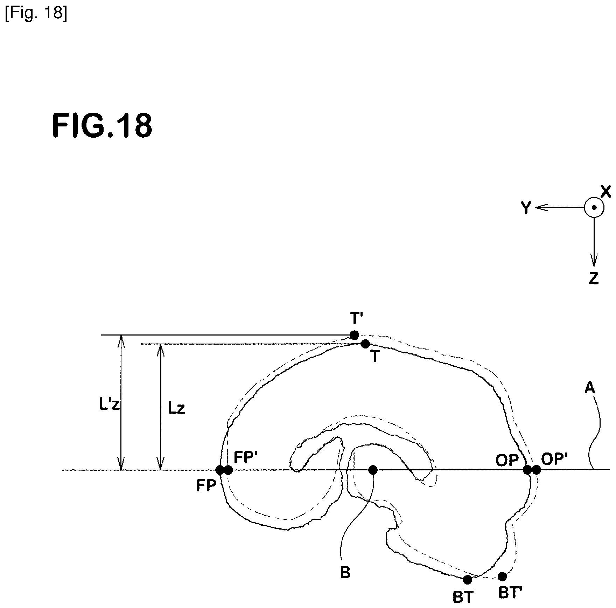

Fig. 18 shows the linear transformation of the bloodstream image as to adjust the bloodstream image with the anatomical standard brain.
[Fig. 19]

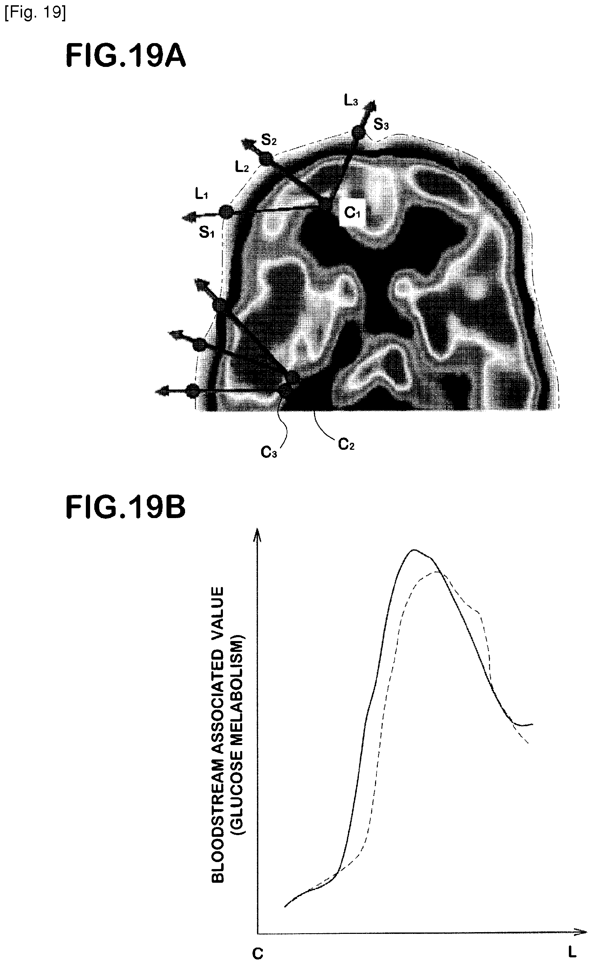

Fig. 19 shows a non-linear transformation of the bloodstream image in accordance with the anatomical standard brain.
[Fig. 20]

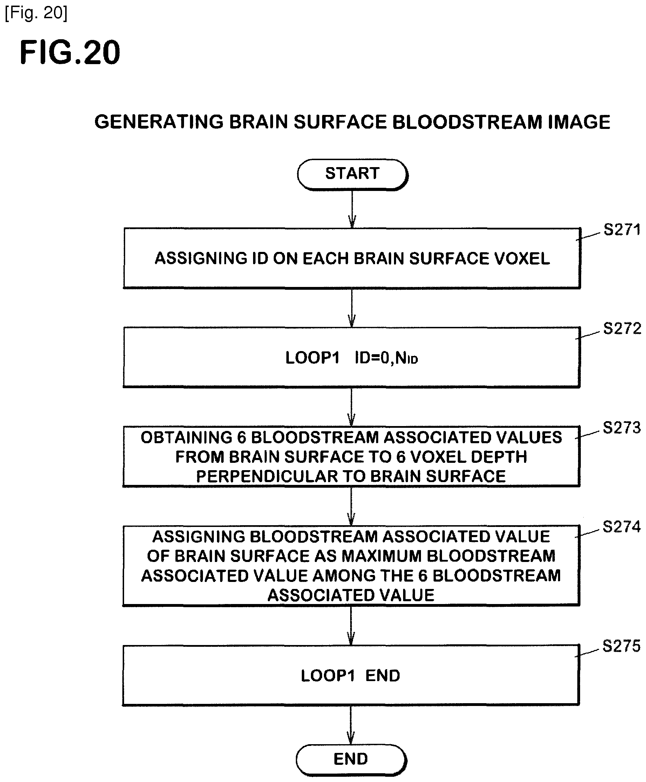

Fig. 20 shows a detailed flowchart for generating the brain bloodstream image (step S27 of Fig.11).
[Fig. 21]

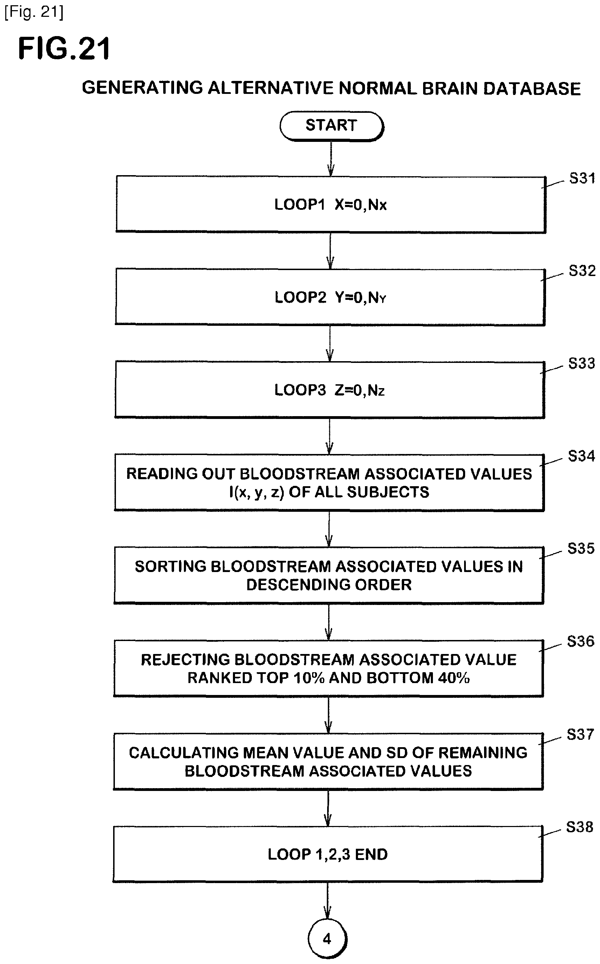

Fig. 21 shows a detailed flowchart for generating the alternative of a normal brain database (step S3 of Fig.6).
[Fig. 22]

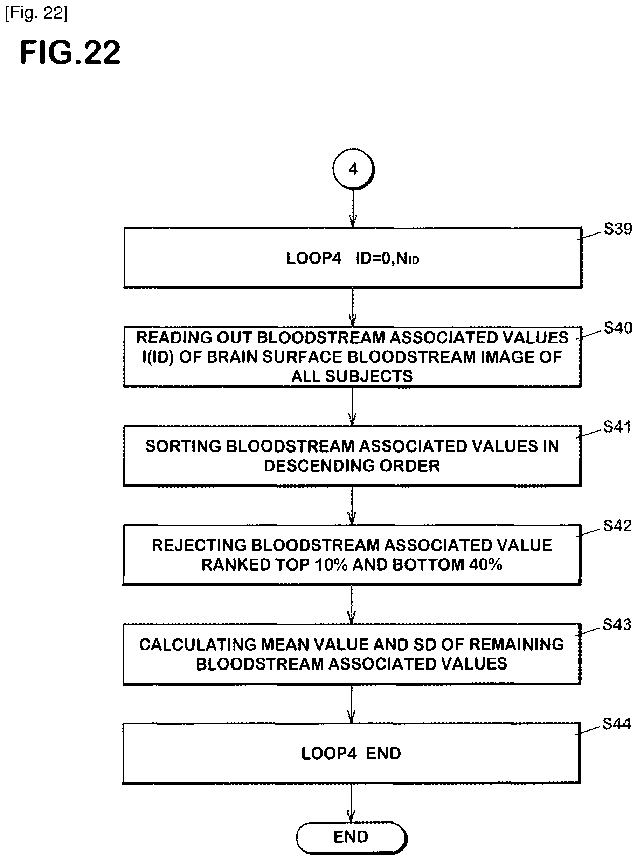

Fig. 22 shows a detailed flowchart for generating the alternative of a normal brain database (step S3 of Fig.6).

**[Fig. 23]**

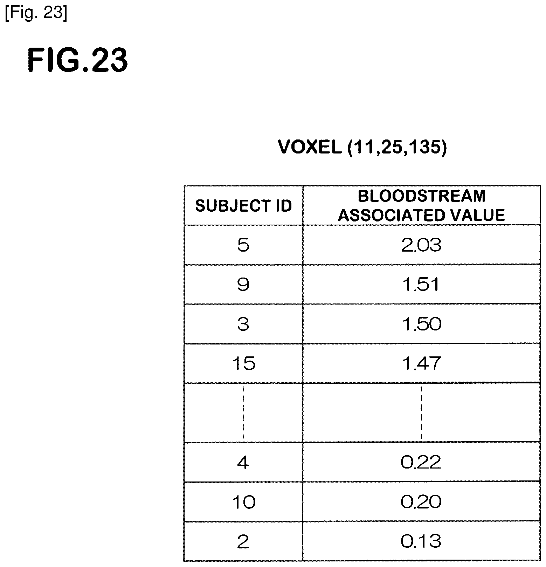

Fig. 23 shows a temporally table for the rejecting bloodstream associated value (s) presumed to indicate disease with regard to a particular voxel.
**[Fig. 24]**

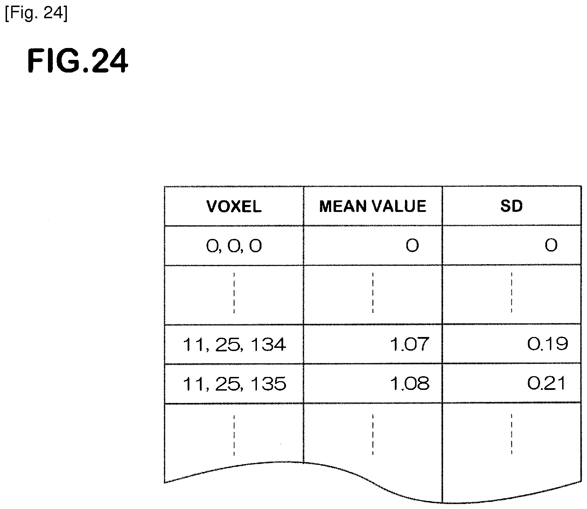

Fig. 24 shows a part of the generated alternative of a normal brain database of brain bloodstream.
**[Fig. 25]**

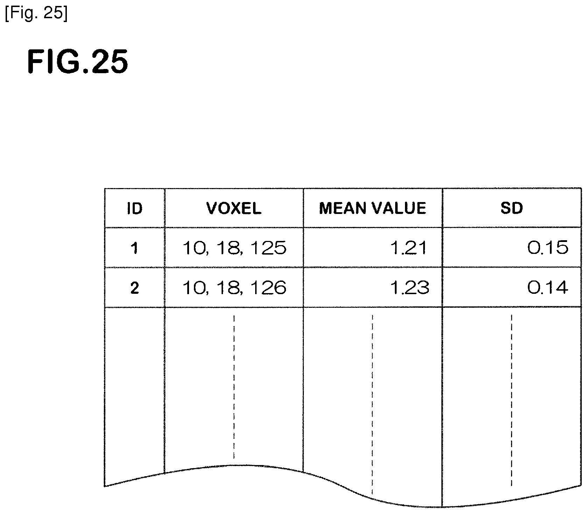

Fig. 25 shows a part of the generated alternative of a normal brain database of a brain surface bloodstream.
**[Fig. 26]**

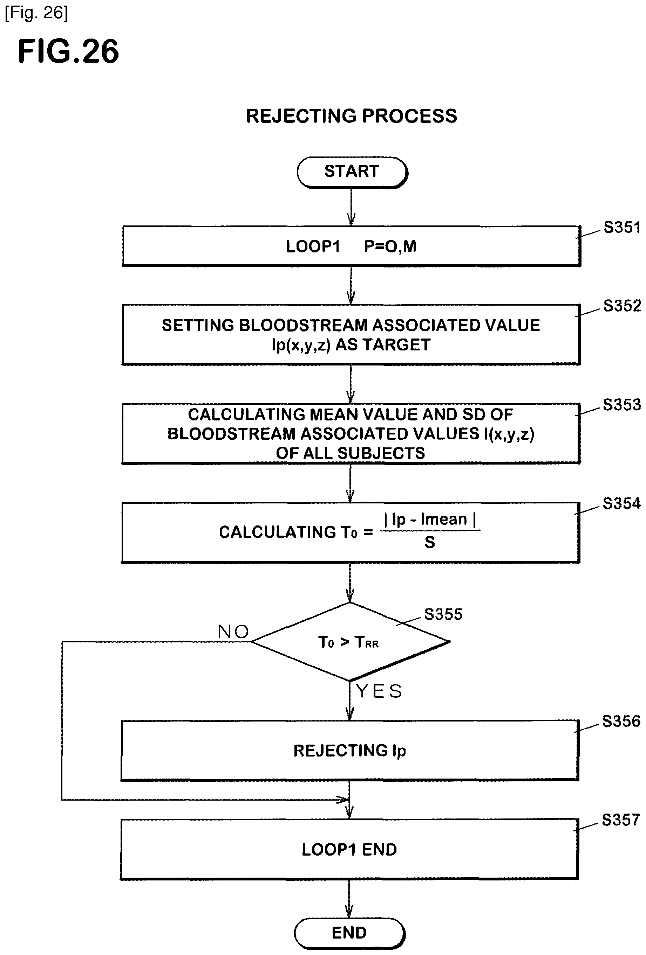

Fig. 26 shows a detailed flowchart of the rejecting process in another embodiment.
**[Fig. 27]**

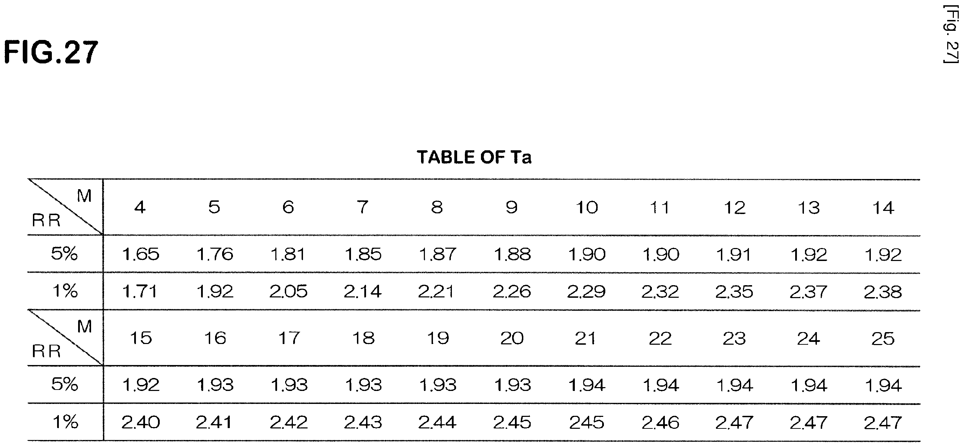

Fig. 27 shows a table of T for the rejection in another embodiment.

**[Description of Embodiments]**

1. Functional Block Diagram

Fig. 3 shows a functional block diagram of a device for generating an alternative of a normal brain database of an embodiment of the present invention. The device is adapted to be able to generate the alternative of a normal brain database based on a plurality of brain status images of each subject including patient(s). All of the plurality of the brain status images provided to the device may be the patient's brain status images. Documents US2007/081699A1 and WO/2008/093057A1 both disclose the generation of normal brain databases. Brain image data is acquired to be compared with brain status images of subjects in order to identify abnormal changes in the brain, indicating a brain disease. The alternative of a normal brain database described herein differs from the above mentioned references by the teaching of rejecting status values presumed to indicate a disease, as will be explained below.

Referring to Fig. 3, an anatomical standardization means 2 obtains the brain status images having brain status values and spatially transforms the brain status images of each subject in accordance with an anatomical standard brain such as Talairach's standard brain so that the brain status images of each subject is standardized by adjusting spatial differences.

The rejecting means 4 identifies the brain status values presumed to indicate disease based on a comparison of the brain status values of subjects with each other at each point of the standardized brain images. Then the rejecting means 4 rejects the brain status values presumed to indicate disease. This rejecting process may be carried out based on a statistical method. In one embodiment, the brain status values of each point are sorted in descending order and the n-th largest and/or the m-th smallest values are rejected. The rejecting means 4 carries out the rejecting process for all of the points.

The database generating means 6 comprises a mean value calculating means 8 and a standard deviation calculating means 10. The mean value calculating means 8 calculates the mean value of the remaining brain status values after the rejecting process with regard to each point. The standard deviation calculating means 10 calculates the standard deviation of remaining brain status values after the rejecting process with regard to each point. The calculated mean value and standard deviation is recorded associated with the information showing the position of each point. The mean values, standard deviations and positions constitute the alternative normal brain database.

Fig. 4 shows a functional block diagram of a device for generating an alternative of a normal brain database in another embodiment of the present invention. The device generates an alternative of a normal brain database of the brain surface status for such as the Three-Dimensional Stereotactic Surface Projection (3D-SSP) method.

Referring to Fig. 4, an anatomical standardization means 2 obtains the brain status images having brain status values and spatially transforms the brain status image of each subject in accordance with anatomical standard brain.

The generating means 3 generates a brain surface status image that has the representative status value of each point of the brain surface which are selected from status values near the brain surface. The generating means 3 generates the brain surface status images of all subjects.

The rejecting means 4 rejects the brain surface status values presumed to indicate disease by comparing the brain surface status values of all subjects with each other at each point of the standardized brain status images.

The database generating means 6 comprises a mean value calculating means 8 and a standard deviation calculating means 10. The mean value calculating means 8 calculates the mean value of the remaining brain surface status values after the rejecting process with regard to each point. The standard deviation calculating means 10 calculates the standard deviation of the remaining brain surface status values after the rejecting process with regard to each point. Each calculated mean value and standard deviation is recorded at each point of the standardized brain status images. The mean values, standard deviations and positions are comprised of the alternative of a normal brain database.

## 2. Hardware construction

Fig. 5 shows the hardware construction of the device of Figs. 3 and 4. Although the device realizes both of the functions shown in Figs. 3 and 4 in the following embodiment, the device may have either function. In the following embodiment, the brain bloodstream associated value is disclosed as an example of brain status value.

CPU12 is connected to a hard-drive drive 14, a CD-ROM drive 16, a display device 18 for displaying brain image etc., a memory 20 and a keyboard/mouse 22. The memory 20 is used as the working area of CPU 12. The keyboard/mouse 22 is used for the inputof instructions from the user.

The hard-drive records an operating system (OS) 24, such as WINDOWS™, a generating program 26 for the alternative of a normal brain database and an anatomical standard brain data 28. These programs and data are installed from a recording medium such as a CD-ROM 30 by using the CD-ROM drive 16. The generating program 26 fulfills its function by cooperating with the OS 24.

## 3. Process by the generating program 26 for a normal database

Fig. 6 is a flowchart of the generating program for an alternative of a normal brain database. The generating process comprises three steps, obtaining brain bloodstream associated values and normalizing (step S1), anatomical standardization (step S2) and generating an alternative of a normal brain database (step S3). Although the brain bloodstream associated value is used in the following embodiment, other functional images (values) may be used.

### 3.1 Obtaining brain bloodstream associated values and normalizing (step S1)

Detailed steps for obtaining and normalizing the brain bloodstream associated values are shown in Fig. 7. First, CPU 12 obtains SPECT data from a plurality of subjects including patient(s) (step S11). The device shown in Fig. 5 can obtain the SPECT data directly from a SPECT device when the device is connected to a SPECT device through a local area network (LAN) etc. The device may obtain the SPECT data by reading the data from the recording medium on which the SPECT device records measured data (SPECT data). The SPECT data comprises projection data obtained from the subject by using a SPECT device.

CPU 12 reconstructs the three-dimensional image data which has bloodstream associated values of, for example, 2 mm cubic voxels based on the SPECT data (step S12).

Then, CPU 12 normalizes the voxel values, because the voxel value of the three-dimensional image is under the influence of measurement condition differences including the measurement device difference. In the normalizing step, CPU 12 divides the bloodstream associated value of each point, i.e. each voxel, by the mean value of the bloodstream associated values of each subject's entire brain (mean value of entire brain voxels) and records the divided values as the normalized bloodstream associated values on the hard-drive 14.

The mean value of the bloodstream associated values of subthalamic, cerebella, pons or sensorimotor cortex may be used as the normalizing standard part instead of the mean value of the bloodstream associated values of the entire brain in the normalizing step. Subthalamic, cerebella, pons or sensorimotor cortex from the SPECT data can be identified by superposing the subject's image on the anatomical standard brain image in which area of each part is predetermined. The normalizing standard part is suitably a part where the reduction of bloodstream associated value is not observed in the disease to be diagnosed.

Fig. 8 shows normalized data of the three-dimensional bloodstream image. In Fig. 8, column "X, Y, Z" represents position data in three-dimensional coordinates, X indicates X-coordinate data, Y indicates Y-coordinate data and Z indicates Z-coordinate data. As shown in Fig. 9, X denotes the horizontal direction, Y denotes the anteroposterior direction and Z denotes the up and down direction of the head. The normalized bloodstream data are generated for each subject and recorded on the hard-drive 14.

### 3.2 Anatomical standardization (step S2)

CPU 12 spatially transforms the normalized three-dimensional bloodstream image of each subject in accordance with an anatomically standard brain (step S2 of Fig. 6). A detailed flowchart of an anatomical standardization is shown in Figs. 10 and 11.

### 3.2.1 Identifying the interhemispheric mid-sagittal plane

CPU 12 reads out the normalized 3-D bloodstream image (see Fig.8) of the initial subject from the hard-drive 14 (step S21). Then, CPU 12 identifies the interhemispheric mid-sagittal plane of the normalized 3-D bloodstream image read out in step S21 (step S22). Referring to Fig. 9, the interhemispheric mid-sagittal plane is defined as the Y-Z plane including line A which passes the center of a head in a horizontal direction: that is, a plane which equally divides the head with regard to the horizontal direction.

In this embodiment, briefly, a method for identifying the interhemispheric mid-sagittal plane is as follows:

First, a center point of the normalized 3-D bloodstream image is identified and a Y-Z plane including the center point is assumed as the interhemispheric mid-sagittal plane. Then, the normalized 3-D bloodstream image is flipped with respect to the assumed interhemispheric mid-sagittal plane as the flipping plane. An image which is symmetric to the assumed interhemispheric mid-sagittal plane is generated. The similarity index between the generated plane symmetry image and the original image (the normalized 3-D bloodstream image) is calculated.

Then, the assumed interhemispheric mid-sagittal plane is moved along the X direction, rotated around the Z axis and rotated around the Y axis. The similarity index is calculated for each assumed interhemispheric mid-sagittal plane.

The interhemispheric mid-sagittal plane is identified by selecting the assumed interhemispheric mid-sagittal plane which has the maximum similarity index, because the similarity index should be at its maximum when the normalized 3-D bloodstream image is flipped at the center plane.

A detailed flowchart for identifying the interhemispheric mid-sagittal plane is shown in Figs. 12 and 13. CPU 12 decides the center point xo, yo, zo of the normalized 3-D bloodstream image by simply using a coordinate position. A new center point is determined by moving the center point along the X direction with dx (step S222).

CPU 12 assumes that the original point of the coordinate is the new center point and rotates the Y-Z plane which passes through the center point around the Z axis with drz and around the Y axis with dry. CPU 12 assumes the moved and rotated plane as the interhemispheric mid-sagittal plane (step S225).

CPU 12 generates a flipped normalized 3-D bloodstream image, which is symmetric to the assumed interhemispheric mid-sagittal plane, by flipping the normalized 3-D bloodstream image (step S226). Then, CPU 12 calculates similarity index between the original and the flipped normalized 3-D bloodstream images (step S227). In this embodiment, Stochastic Sign Change (SSC) is calculated as the similarity index.

The concept of calculating the SSC is as follows:

Consider two similar but not identical images $I_1(x, y)$ and $I_2(x, y)$, where $I(x, y)$ is the pixel count and x, y = 1, 2, .... n are the coordinates of the digitized images. Let $S(x, y) = I_1(x, y) - I_2(x, y)$ be the subtraction image. If $I_1(x, y)$ and $I_2(x, y)$ contain additive noise which can be assumed to have a zero mean with a symmetric density function, each pixel value of $S(x, y)$ is not zero but shows random fluctuations around zero, either positive or negative values with equal probability. If there is a dissimilar part of the images between $I_1(x, y)$ and $I_2(x, y)$, the pixel values of $S(x, y)$ in that part will no longer exhibit random fluctuations and will show groupings of all positive or negative values. Let SSC represent the number of sign changes in a sequence of the $S(x, y)$, scanned line-by-line or column-by-column. Accordingly, SSC shows a larger number of sign changes when $I_1(x, y)$ and $I_2(x, y)$ are similar and a lower value when $I_1(x, y)$ and $I_2(x, y)$ are dissimilar. Therefore, the SSC criterion can be defined as a similarity criterion between two images. This concept can be applied on judging the similarity between two three-dimensional images.

SSC is calculated by the summation of $SSC_x$, $SSC_y$ and $SSC_z$ in this embodiment. Referring to Fig. 14, planes $ZX_1, ZX_2, .... ZX_N$ and planes $YX_1, YX_2, .... YX_N$ are assumed for calculating $SSC_x$. For each plane, SSC is calculated by scanning the plane. $SSC_x$ is calculated by the summation of SSC of all planes $ZX_1, ZX_2, .... ZX_N$ and $YX_1, YX_2, .... YX_N$. CPU 12 also calculates $SSC_y$ by the summation of SSC of planes $ZY_1, ZY_2, .... ZY_N$ and planes $YX_1, YX_2, .... YX_N$ and $SSC_z$ by the summation of SSC of planes $ZY_1, ZY_2, .... ZY_N$ and planes $ZX_1, ZX_2, .... ZX_N$. Then, CPU 12 obtains SSC(dx, drz, dry) with regard to the assumed interhemispheric mid-sagittal plane as the summation of $SSC_x$, $SSC_y$ and $SSC_z$ and stores SSC(dx, drz, dry) on the memory 20.

CPU 12 changes dx from -I to I at 1 voxel step to move the assumed interhemispheric mid-sagittal plane along the X axis and changes drz and dry from -rI to rI to rotate the assumed interhemispheric mid-sagittal plane. CPU 12 calculates SSC(dx, drz, dry) for all assumed interhemispheric mid-sagittal planes which are determined by all combinations of dx, drz and dry (step S221, S223 and S224).

After calculating SSC(dx, drz, dry) of all the assumed interhemispheric mid-sagittal planes, CPU 12 identifies the interhemispheric mid-sagittal plane which has the largest SSC(dx, drz, dry) among all of the assumed interhemispheric mid-sagittal planes (step S229). Identifying the interhemispheric mid-sagittal plane is disclosed in Minoshima et al., "An Automated Method for Rotational Correction and Centering of Three-Dimensional Functional Brain Images" J Nucl Med 1992; 33: 1579-1585, which is expressly incorporated by reference herein.

### 3.2.2 Identifying the AC-PC line and the center thereof

After identifying the interhemispheric mid-sagittal plane, CPU 12 identifies the line which passes through the anterior and the posterior commissures of the brain (herein after referred to as the "AC-PC line") and the center thereof (step S23 of Fig. 10). In this embodiment, 4 landmarks, occipital pole point (OP), the subthalamic point (TH), the inferior aspect of the anterior corps callosum (CC) and the frontal pole point (FP) of the brain are decided and then the AC-PC line is identified as a straight line connecting the 4 landmarks.

A detailed flowchart for identifying the AC-PC line and the center thereof is shown in Fig. 15. CPU 12 reads out the sectional bloodstream image at the interhemispheric mid-sagittal plane from the hard-drive 14. CPU 12 further reads out the sectional bloodstream images of the planes which are parallel to and near the interhemispheric mid-sagittal plane (step S231). Fig. 16A shows an example of the sectional bloodstream image at the interhemispheric mid-sagittal plane.

The CPU 12 detects the outer border of the brain and the border between white matter and gray matter of the brain for each normalized 3-D bloodstream image (step S232). The detection can be carried out by detecting the border between the area where the bloodstream is existing and the area where the bloodstream is non-existing. Fig. 16B shows a detected border image of the interhemispheric mid-sagittal plane for example.

Then, CPU 12 identifies the occipital pole point (OP) as the most posterior point of the border image of the interhemispheric mid-sagittal plane (see Fig. 16B). CPU 12 also identifies the occipital pole points (OPs) for the other border images of the other sectional bloodstream images. CPU 12 calculates the mean of the y coordinate values and the mean of the z coordinate values of all of the identified occipital pole points (OPs). CPU 12 decides the position of the occipital pole point (OP) based on the mean of y coordinate values and the mean of z coordinate values on the interhemispheric mid-sagittal plane (step S233).

Next, CPU 12 finds out the U shaped border area as shown in Fig. 16C. The inferior aspect of the anterior corps callosum (CC) is decided by detecting a contact point of the U shaped border and a tangent line from the occipital pole point (OP) to the U shaped border based on the border image. CPU 12 decides the inferior aspect of the anterior corps callosum (CC) for each border image of the sectional bloodstream image. CPU 12 calculates the mean of the y coordinate values and the mean of the z coordinate values of all of the found inferior aspects of the anterior corps callosum (CC). CPU 12 decides the position of the inferior aspects of the anterior corps callosum (CC) based on the mean of the y coordinate values and the mean of the z coordinate values on the interhemispheric mid-sagittal plane (step S234).

CPU 12 identifies the frontal pole point (FP) as the most anterior point of the border image of the interhemispheric mid-sagittal plane (see Fig. 16B). CPU 12 also identifies the frontal pole points (FPs) based on the other border images of the other sectional bloodstream images. CPU 12 calculates the mean of the y coordinate values and the mean of the z coordinate values of all the identified frontal pole points (FPs). CPU 12 decides the position of the frontal pole point (FP) based on the mean of the y coordinate values and the mean of the z coordinate values on the interhemispheric mid-sagittal plane (step S235).

CPU 12 decides the subthalamic point (TH) (step S236). To decide the subthalamic point (TH), CPU 12 at first finds the thalamic center. The thalamic center can be estimated as the point having a local maximum bloodstream associated value near the AC-PC line. In this embodiment, CPU 12 finds a point having the local maximum bloodstream associated value within the circle of a radius r on the sectional bloodstream image and identifies the point as the thalamic center, where the center of the circle is a center point of the line connecting the occipital pole point (OP) with the frontal pole point (FP) and where the radius r is a tenth of the length between OP and FP.

After deciding the thalamic center, CPU 12 plots an imaginary circle having a predetermined radius r on the sectional bloodstream image. As shown in Fig. 17A, CPU 12 makes the imaginary point P1 where the imaginary circle and the tangent line from OP contact the imaginary circle and the imaginary point P2 which is diametrically opposite to P1 on the imaginary circle. CPU 12 stores the bloodstream associated values on each imaginary point P2 of the imaginary circle on the memory 20 when the radius r of the imaginary circle is varied from the length of 1 pixel to 12 pixels by a 0.5 pixel step. Fig. 17B shows a graph plotting the bloodstream associated values on the imaginary point P2 at each radius r. In this graph, the horizontal axis denotes the radius r and r=0 is positioned at the center. To the left and right sides from the center, r is increased. The graph shows that the bloodstream associated value becomes at maximum when the radius r is 0. Increasing r makes the bloodstream associated value on the imaginary point P2 reduced towards the minimum bloodstream associated value which corresponds to the lateral ventricular (LV).

CPU 12 decides a radius r which corresponds to the border of thalamus at which the bloodstream associated value on the imaginary point P2 shows 70% of the maximum bloodstream associated value where the minimum bloodstream associated value is set as 0% (see Fig. 17). Then, CPU 12 identifies the imaginary point P1 on the imaginary circle having the decided radius r as the subthalamic point (TH). CPU 12 also decides the subthalamic point (TH) based on the other sectional bloodstream images. CPU 12 calculates the mean of the y coordinate values and the mean of the z coordinate values of all of the decided subthalamic points (THs). CPU 12 decides the position of the subthalamic point (TH) based on the mean of y coordinate values and the mean of the z coordinate values on the interhemispheric mid-sagittal plane (see Fig. 16B).

CPU 12 generates a regression line connecting the occipital pole point (OP), the subthalamic point (TH), the inferior aspect of the anterior corps callosum (CC) and the frontal pole point (FP) on the interhemispheric mid-sagittal plane. CPU 12 identifies the generated regression line as the AC-PC line A (step S237). Then, the center point B of the AC-PC line A is decided as a point which possesses half length of the AC-PC line as shown in Fig. 16D (step S238). Identifying the AC-PC line and the center thereof is disclosed in Minoshima et al., "An Automated Detection of the Intercommissural Line for Stereotactic Localization of Functional Brain Images" J Nucl Med 1993; 34: 322-329, which is expressly incorporated herein by reference.

### 3.2.3 Positioning the subject's image to a normal brain

Once the AC-PC line and the center thereof are identified, CPU 12 positions the normalized 3-D bloodstream image of each subject to the standard brain image data 28 recorded in the hard-drive 14 by superposing the AC-PC lines and the centers of both images (step S24 of Fig. 10). The direction of the subject's normalized 3-D bloodstream image is aligned to that of the standard brain image by matching the AC-PC line of the subject's normalized 3-D bloodstream image to that of the standard brain image. The position of the subject's normalized 3-D bloodstream image is aligned to that of the standard brain image in the anteroposterior direction by matching the center of the AC-PC line to that of the standard brain image.

In this embodiment, a $^{18}$F-FDG PET image is used as the standard brain image. The standard image is made by transforming the images of a number of normal subjects in accordance with the standard brain shape and averaging them. The standard brain image of $^{18}$F-FDG PET is well used and easily obtainable.

### 3.2.4 Linearly transforming the subject's image

After positioning the subject's normalized 3-D bloodstream image to the normal brain, CPU 12 linearly transforms the subject's normalized 3-D bloodstream image in accordance with the standard brain image by the following steps.

First, CPU 12 provides a Y-axis which is the AC-PC line of the subject's normalized 3-D bloodstream image, a Z-axis which is the line on the interhemispheric mid-sagittal plane which passes through the center of the AC-PC line and is perpendicular to the AC-PC line and a X-axis which is the normal line of the interhemispheric mid-sagittal plane which passes through the center of the AC-PC line. Therefor, the center of the AC-PC line is provided as the original point of the coordinate system. Then, CPU 12 obtains Y coordinate positions of the most anterior and the most posterior points, Z coordinate positions of the most upper and the lowest points and X coordinate positions of the most right and the most left points of the subject's normalized 3-D bloodstream image. CPU 12 also obtains the Y coordinate positions, the Z coordinate positions and the X coordinate positions of the standard brain image which may be previously obtained and stored.

CPU 12 transforms the subject's normalized 3-D bloodstream image so that the outer border of the subject's bloodstream image is adjusted to the outer border of the standard brain image by using the obtained X, Y and Z coordinate positions of the subject's normalized 3-D bloodstream image and shoes of the standard brain image (step S25 of Fig. 10).

Referring to Fig. 18, for example, the subject's normalized 3-D bloodstream image (shown by solid line) is superposed on the standard brain image (shown by chained line). CPU 12 finds the most upper point T of the outer border of the subject's normalized 3-D bloodstream image and obtains the Z coordinate value Lz of the point T. CPU 12 also finds the most upper point T' of the outer border of the standard brain image and obtains the Z coordinate value L'z of the point T'. Then, CPU 12 transforms the Z coordinate values of the subject's normalized 3-D bloodstream image by the following equation:

$$Z' = Z(Lz / L'z)$$

where Z' is the transformed Z coordinate value of the subject's normalized 3-D bloodstream image, Z is the original Z coordinate value of the subject's normalized 3-D bloodstream image, Lz is the Z coordinate value of the point T and L'z is the Z coordinate value of the point T'.

By the transformation, the subject's normalized 3-D bloodstream image is adjusted to the standard brain image with respect to the upper direction.

CPU 12 also transforms the subject's normalized 3-D bloodstream image with respect to lower, right, left, anterior and posterior direction in the same way as that of the upper direction.

### 3.2.5 Non-linearly transforming the subject's image

Although the subject's normalized 3-D bloodstream image is aligned in accordance with the standard brain image by the linear transforming, some misalignments between the two images still remain. CPU 12 partially transforms the subject's normalized 3-D bloodstream image based on the profile curve of the bloodstream associated value of the subject's normalized 3-D bloodstream image and the profile curve of the glucose metabolism of the standard brain image (step S26).

Referring to Fig. 19A, a plurality of landmarks C1, C2 .... Cn on white matter are predetermined in the standard brain image. A plurality of landmarks S1, S2 .... Sn on the brain surface are also predetermined corresponding to the landmarks C1, C2 .... Cn in the standard brain image. In Fig. 19A, the landmarks S1, S2 and S3 of the brain surface are provided corresponding to the landmarks C1 of white matter. CPU 12 generates the profile curve of the bloodstream associated values by obtaining the bloodstream associated values on the subject's normalized 3-D bloodstream image along a line L1 connecting the landmarks from C1 to S1 (see solid curve of Fig. 19B). CPU 12 obtains the profile curve of glucose metabolism on the standard brain image along the line L1 (see chained curve of Fig. 19B). In this embodiment, the profile curve of glucose metabolism on the standard brain image is prerecorded in the hard-drive 14.

CPU 12 partially transforms the subject's normalized 3-D bloodstream image so that the profile curve of the bloodstream associated values of the subject's normalized 3-D bloodstream image accords to the profile curve of the glucose metabolism values at each corresponding point, since the profile curve of the glucose metabolism values is well matched with that of the bloodstream associated values. The partial transformation (non-linear transformation) is carried out along the direction of line L1 and the landmark C1 is fixed. With regard to the line L2 between the landmarks C1 and S2 and the line L3 between the landmarks C1 and S3, CPU 12 also transforms the subject's normalized 3-D bloodstream image in the same way. In the area between the lines L1 and L2 (L2 and L3), CPU 12 transforms these areas based on the transforming rates on L1 and L2 (L2 and L3). The non-linear transformation is carried out with regard to each line L connecting each landmark C of white matter and corresponding landmark S of the brain surface. In the above description, non-linear transformation is described on a two-dimensional model, however in this embodiment, the non-linear transformation is carried out in three-dimensional space.

The linear and non-linear transforming is disclosed in Minoshima et. al. "An Anatomic Standardization: Linear Scaling and Nonlinear Warping of Functional Brain Images" J Nucl Med 1994; 35: 1528-1537, which is expressly incorporated by reference herein.

### 3.2.6 Generating the brain surface bloodstream image

After anatomically transforming the subject's transformed 3-D bloodstream image, CPU 12 generates the brain surface bloodstream image (step S27 of Fig. 11). Fig. 20 shows a detailed flowchart for generating the brain surface bloodstream image.

CPU 12 identifies each brain surface voxel positioned at the most outer surface of the subject's normalized 3-D bloodstream image and assigns an identification number for each identified surface voxel (step S271). In this embodiment, approximately 20,000 surface voxels are identified.

CPU 12 carries out the following steps for each identified surface voxel:
CPU 12 obtains the bloodstream associated values of several number ("6" in this embodiment) of voxels from the brain surface to a predetermined depth perpendicular to the brain surface in the subject's transformed 3-D bloodstream image (step S273). Then, CPU 12 selects the maximum bloodstream associated value among the several numbers of voxels as a representative bloodstream associated value and the selected maximum bloodstream associated value is assigned to the brain surface voxel (step S274).

CPU 12 carries out such process for each brain surface voxel. Then, a brain surface bloodstream image in which the representative bloodstream associated value is indicated at each surface voxel is obtained.

CPU 12 stores the brain bloodstream images generated in step S26 and the brain surface bloodstream images generated in step S27 on the hard-drive 14 (step S28 of Fig. 11).

When the next subject's unprocessed bloodstream image exists, CPU 12 reads out the next subject's unprocessed bloodstream image from the hard-drive 14 (step S30) and carries out the process described above (following steps of step S22 of Fig. 10). When all of the subject's bloodstream images are processed (step S29), CPU 12 finishes the anatomical standardization (step S2 of Fig. 6).

### 3.3 Generating an alternative of a normal brain database

After generating an anatomically standardized subject's brain bloodstream image and a brain surface bloodstream image, CPU 12 generates an alternative of a normal brain database (step S3 of Fig. 6). When all subjects are normal and healthy, the alternative of a normal brain database can simply be obtained by calculating the mean value and standard deviation of each point of the anatomically standardized brain bloodstream images and brain surface bloodstream images. However, in this embodiment the subjects contain patient(s). Therefore, in this embodiment, the alternative of a normal brain database is generated after the rejection of bloodstream associated values regarded as disease part.

Figs. 21 and 22 show detailed flowcharts for generating the alternative of a normal brain database. CPU 12 reads out the normalized bloodstream associated values of target voxel of all of the subject's standardized brain bloodstream images (step S34). Then, CPU 12 sorts the bloodstream associated values in descending order (step S35). Fig. 23 shows sorted bloodstream associated values of the target voxel. In Fig. 23, the bloodstream associated values of the subjects at a target voxel (positioned at x=11, y=25 and z=135) are shown in descending order. Subject ID means the identification of the subject, which is assigned to each subject uniquely.

The CPU 12 rejects bloodstream associated values that are ranked in the top 10% and in the bottom 40% (step S36). The percentage of rejection may be selected arbitrarily. When the number of subjects are 20 for example, bloodstream associated values of the highest 2 subjects and of the lowest 8 subjects are rejected. In this embodiment, bottom values are rejected, because a part having a reduced bloodstream associated value is regarded as corresponding to a disease part such as Alzheimer's disease. Higher values are also rejected, because it sometimes occurs that even the bloodstream associated value of the part except the disease part vary widely from those of the normal healthy subjects due to the influence of the normalization (step S13 of Fig. 7). Because the lower values are regarded as corresponding to the disease part, rejected percentages of the lower values are higher than that of the higher values in this embodiment.

Then, the CPU 12 calculates and stores mean values and standard deviations of the remaining bloodstream associated values after the rejection (step S37). As described above, mean values and standard deviations of the bloodstream associated values at target voxel (11, 25, 135) are calculated and stored on the hard-drive 14 (see Fig. 24).

CPU 12 calculates mean values and standard deviations of the bloodstream associated values for each voxel as for a target voxel (steps S31, S32, S33 and S38). Then, the alternative of a normal brain database of brain bloodstream image is obtained as shown in Fig. 24.

Next, the CPU 12 calculates mean values and standard deviations of the bloodstream associated values with regard to the patient's brain surface bloodstream images. CPU 12 reads out the bloodstream associated values of target surface voxel of all of the subject's standardized brain bloodstream images (step S40). Then, the CPU 12 sorts the bloodstream associated values in descending order (step S41).

CPU 12 rejects the bloodstream associated values that are ranked in the top 10% and in the bottom 40% (step S42). When the number of the subjects are 20 for example, the bloodstream associated values of the highest 2 subjects and of the lowest 8 subjects are rejected.

Then, the CPU 12 calculates and stores mean values and standard deviations of the remaining bloodstream associated values after the rejection (step S43). The alternative of a normal brain database of brain surface bloodstream image is obtained as shown in Fig. 25.

_4 Other embodiments_

Identifying interhemispheric mid-sagittal plane and PC-AC line and positioning AC-PC line shown as steps S22, S23 and S24 of Fig. 10 may be substituted by aligning method using mutual information in which the subject's bloodstream image is moved against the standard brain image in dribs and mutual information between the both images is used to find the best aligning position. The aligning method using mutual information is disclosed in F. Maes et al., "Multimodality Image Registration by Maximization of Mutual Information," IEEE Transactions on Medical Imaging, (USA), 1997, 16, 2, p187-198, which is expressly incorporated herein by reference. The aligning method using mutual information can be achieved by the stereo program contained in 3D-SSP program (provided by Satoshi Minoshima, Professor of University of Washington).

The bloodstream associated values regarded as outliers due to the normalization are rejected in addition to the bloodstream associated values regarded as brain disease part in the above mentioned embodiment. However, only one of the bloodstream associated values regarded as an outlier due to the normalization or the bloodstream associated values regarded as brain disease part may be rejected.

It is suitable to assume the bloodstream associated values indicating disease part and to reject the predetermined percentage of the lower bloodstream associated values as described in the above mentioned embodiment, when the number of subjects is large and the predetermined percentage can be accurately predicted, because the rejection can be carried out by simple calculation.

Instead of the above mentioned rejection method, following statistical rejection methods may be used. For example, THOMPSON's method (William R. Thompson, The annals of Mathematical Statistics, Vol 6, No.4(Dec 1935), pp214-219), SMIRNOV-GRUBBS's method (Frank E. Grubbs, The annals of Mathematical Statistics, Vol 21, No. 1 (Mar., 1950), pp.27-58) may be used.

When using THOMPSON's method, steps S35 and S36 of Fig. 21 are substituted for steps shown in Fig. 26.

Referring to Fig. 26, the CPU 12 sets a bloodstream associated value Ip (x, y, z) of a subject as the targeted subject's bloodstream associated value (step S352). CPU 12 calculates the mean value $I_{mean}$ (x, y, z) and the standard deviation SD (x, y, z) of the bloodstream associated values of all subjects (step S353). Then, the CPU 12 calculates the index To according to the following equation (step S354):

$$T_0 = (Ip\ (x,\ y,\ z) - I_{mean}(x,\ y,\ z))\ /\ SD(x,\ y,\ z)$$

CPU 12 compares the index To and limits Ta (step S355). The limit Ta is decided by the combination of the risk rate RR and the number of subjects M. In this embodiment, the CPU 12 obtains the limit Ta according to a table (prerecorded on the hard-drive 14) as shown in Fig. 27. The risk rate RR is a factor indicating the rate of rejection and more bloodstream associated values are rejected when the risk rate RR grows higher. When the number M of the subject is 20 and the risk rate RR is 5%, the limit Ta is decided as 1.93 from the table of Fig. 27.

CPU 12 rejects the targeted subject's bloodstream associated value Ip (x, y, z) when the index To is larger than the limit Ta (step S35) and does not reject the targeted subject's bloodstream associated value Ip (x, y, z) when the index To is not larger than the limit Ta.

CPU 12 repeatedly carries out the above mentioned process for each subject's bloodstream associated value with said value as the target bloodstream associated value (steps S351 and S357).

In the embodiment shown in Fig. 26, the same risk rate RR is used for both the upper side and the lower side outlier. However, the risk rate RR of one side on which the disease affects the bloodstream (for example, the lower bloodstream associated value side) may be higher than that of the other side on which the disease does not affect the bloodstream (for example, the higher bloodstream associated value side). Instead, the rejection process may be carried out only for the one side in which the disease affects the bloodstream.

As shown in the table of Fig. 27, the number of subjects should be more than 3 for the rejecting process. In order to secure the degrees of freedom, it is necessary to have a predetermined number of subjects for the statistical rejecting process, such as Thompson's method.

Although brain bloodstream image obtained by SPECT and PET etc. is used in the above mentioned embodiments, other functional images may be used.

Although normalization of status values (bloodstream associated values) is carried out before the spatial transformation in the above mentioned embodiments, the normalization may be carried out after the spatial transformation or the normalization and the spatial transformation may be carried out simultaneously.

## [ABSTRACT]

CPU 12 reads out the bloodstream associated values of the target voxel of subject's standardized brain bloodstream images (step S34). CPU 12 sorts the bloodstream associated values in descending order (step S35). CPU 12 rejects bloodstream associated values that are ranked as the top 10% and bottom 40% (step S36). When the subjects are 20 for example, bloodstream associated values of the highest 2 subjects and of the lowest 8 subjects are rejected. CPU 12 calculates and stores mean values and standard deviations of the remaining bloodstream associated values after the rejection (step S37). CPU 12 calculates mean values and standard deviations of the bloodstream associated values for each voxel as for the target voxel (steps S31, S32, S33 and S38). Then, the alternative normal brain database of brain bloodstream image is obtained.

## [DRAWINGS]

[Fig. 1]
[Fig. 2]
[Fig. 3]
[Fig. 4]
[Fig. 5]
[Fig. 6]
[Fig. 7]
[Fig. 8]
[Fig. 9]
[Fig. 10]
[Fig. 11]
[Fig. 12]
[Fig. 13]
[Fig. 14]
[Fig. 15]
[Fig. 16]
[Fig. 17]
[Fig. 18]
[Fig. 19]

**[Fig. 20]**
**[Fig. 21]**
**[Fig. 22]**
**[Fig. 23]**
**[Fig. 24]**
**[Fig. 25]**
**[Fig. 26]**
**[Fig. 27]**

**Claims**

1. A device for generating an alternative of a normal brain database to be used for diagnosing a brain disease based on a plurality of brain status images of each subject from a set of subjects including patients, the alternative of a normal brain database showing normal status of each point of a brain, the device comprising:

   means for normalizing a status value at each point of each brain status image of a subject based on the status values of a part or whole of the brain status image of the subject;
   means for spatially transforming each brain status image of the subject in accordance with an anatomical standard brain;
   means for rejecting the status values from the smallest value to the m-th smallest value and values from the largest value to the n-th largest value presumed to indicate a disease with regard to each point of the brain, when said points are to be used for diagnosing a disease based on said anatomically transformed and normalized brain status images of the subject, wherein the rejecting means identifies the brain status values presumed to indicate disease based on a comparison of the brain status values of subjects with each other at each point of the standardized brain images;
   and
   means for generating the alternative of a normal brain database by obtaining at least a mean status value for said each point of the brain to be used for diagnosing a disease based on a respective point on each subject's said anatomically transformed and normalized brain status images of in which said status values presumed to indicate disease are rejected, wherein a mean value calculating means calculates the mean value of the remaining brain status values.

2. A device for generating an alternative of a normal brain database in accordance with claim 1, wherein said rejecting means rejects the extreme deviate brain status values, statistically obtained at each point, when said extreme deviate brain status values are presumed to indicate a disease.

3. A device for generating an alternative of a normal brain database in accordance with claims 1 or 2, wherein said brain status image is a brain bloodstream image which shows the bloodstream associated value of each point of the brain.

4. A device for generating an alternative of a normal brain database in accordance with claim 1, wherein the number m is larger than the number n

5. A device for generating an alternative of a normal brain database in accordance with any one of claims 1 to 4, wherein said generating means generates the alternative of a normal brain database by obtaining a mean status value and a standard deviation for said each point of the brain, to be used for diagnosing disease based on said anatomically transformed and normalized brain status images of subjects in which said status values presumed as indicating disease are rejected.

6. A device for generating an alternative of a normal brain database in accordance with any one of claims 1 to 5, further comprising:

   means for generating a brain surface status image of each subject based on said anatomically transformed and normalized brain status images of the subjects, said brain surface status image having a surface status value of each surface portion, said surface status value being selected as a representative value of the status values from the brain surface to a predetermined depth perpendicular to the brain surface; and
   wherein said each surface point at which said surface status value is indicated is used as said point of the brain

to be used for diagnosing a disease.

**7.** A computer program for generating an alternative of a normal brain database to be used for diagnosing a brain disease based on the brain status images of each subject from a set of subjects including patients, the alternative of a normal brain database showing normal status of each point of the brain, the program comprising instructions for the steps of:

normalizing the status value at each point of each brain status image of a subject based on the status values of a part or whole of the brain status image of the subject;
spatially transforming each brain status image of the subject in accordance with an anatomical standard brain;
rejecting the status values from the smallest value to the m-th smallest value and values from the largest value to the n-th largest value presumed to indicate a disease with regard to each point of the brain, when said points are to be used for diagnosing a disease based on said anatomically transformed and normalized brain status images of the subject, wherein the rejecting means identifies the brain status values presumed to indicate disease based on a comparison of the brain status values of subjects with each other at each point of the standardized brain images;
and
generating the alternative of a normal brain database by obtaining at least a mean status value for said each point of the brain to be used for diagnosing a disease based on a respective point on each subject's said anatomically transformed and normalized brain status images of in which said status values presumed to indicate disease are rejected, wherein a mean value calculating means calculates the mean value of the remaining brain status values.

**8.** A computer program for generating an alternative of a normal brain database in accordance with claim 7, wherein said rejected status values in said rejecting step are extreme deviate values statistically obtained at each point as said status values are presumed to indicate a disease.

**9.** A computer program for generating an alternative of a normal brain database in accordance with claims 7 or 8, wherein said brain status image is a brain bloodstream image which shows a bloodstream associated value of each point of the brain.

**10.** A computer program for generating an alternative of a normal brain database in accordance with claim 9, wherein the number m is larger than the number n.

**11.** A computer program for generating an alternative of a normal brain database in accordance with any one of claims 7 to 10, wherein
in said generating step the alternative of normal brain database is generated by obtaining a mean status value and a standard deviation for said each point of the brain to be used for diagnosing a disease based on said anatomically transformed and normalized brain status images of the subjects in which said status values are presumed to indicate a disease are rejected.

**12.** A computer program for generating an alternative of a normal brain database in accordance with any one of claims 7 to 11, further comprising instruction for the step of:

generating a brain surface status image of each subject based on said anatomically transformed and normalized brain status images of the subjects, said brain surface status image having a surface status value of each surface portion, said surface status value being selected as a representative value of the status values from the brain surface to a predetermined depth perpendicular to the brain surface; and
wherein said each surface point at which said surface status value is indicated is used as said point of brain to be used for diagnosing a disease.

**13.** A method for generating an alternative of a normal brain database to be used for diagnosing a brain disease based on brain status images of each subject from a set of subjects including patients, the alternative of a normal brain database showing normal status of each point of the brain, the method comprising the steps of:

normalizing a status value at each point of each brain status image of a subject based on a status values of a part or whole of brain status image of the subject;
spatially transforming each brain status image of the subject in accordance with an anatomical standard brain;

rejecting the status values from the smallest value to the m-th smallest value and values from the largest value to the n-th largest value presumed to indicate a disease with regard to each point of the brain, when said points are to be used for diagnosing a disease based on said anatomically transformed and normalized brain status images of the subject, wherein the rejecting means identifies the brain status values presumed to indicate disease based on a comparison of the brain status values of subjects with each other at each point of the standardized brain images;
and
generating the alternative of a normal brain database by obtaining at least a mean status value for said each point of the brain to be used for diagnosing a disease based on a respective point on each subject's said anatomically transformed and normalized brain status images of in which said status values presumed to indicate disease are rejected, wherein a mean value calculating means calculates the mean value of the remaining brain status values.

14. A method for generating an alternative of a normal brain database in accordance with claim 13, wherein said rejected status values in said rejecting step are extreme deviate values statistically obtained at each point as said status values are presumed to indicate a disease.

15. A method for generating an alternative of a normal brain database in accordance with claims 13 or 14, wherein said brain status image is a brain bloodstream image which shows a bloodstream associated value of each point of the brain.

16. A method for generating an alternative of a normal brain database in accordance with claim 13, wherein the number m is larger than the number n.

17. A method for generating an alternative of a normal brain database in accordance with any one of claims 13 to 16, wherein
in said generating step the alternative of a normal brain database is generated by obtaining a mean status value and a standard deviation for said each point of the brain to be used for diagnosing a disease based on said anatomically transformed and normalized brain status images of the subjects in which said status values are presumed to indicate a disease are rejected.

18. A method for generating an alternative of a normal brain database in accordance with any one of claims 13 to 17, further comprising the step of:

generating a brain surface status image of each subject based on said anatomically transformed and normalized brain status images of the subjects, said brain surface status image having a surface status value of each surface portion, said surface status value being selected as a representative value of status values from the brain surface to a predetermined depth perpendicular to the brain surface; and
wherein said each surface point at which said surface status value is indicated is used as said point of the brain to be used for diagnosing a disease.

**Patentansprüche**

1. Vorrichtung zum Erzeugen einer Alternative zu einer normalen Gehirndatenbank, die zu verwenden ist, um eine Gehirnerkrankung basierend auf einer Vielzahl von Gehirnstatusbildern jedes Probanden aus einer Reihe von Probanden, die Patienten umfassen, zu diagnostizieren, wobei die Alternative zu einer normalen Gehirndatenbank einen normalen Status von jedem Punkt eines Gehirns anzeigt, wobei die Vorrichtung aufweist:

Mittel zum Normalisieren eines Statuswertes an jedem Punkt jedes Gehirnstatusbildes eines Probanden basierend auf den Statuswerten eines Teils oder Ganzen des Gehirnstatusbildes des Probanden;
Mittel zum räumlichen Transformieren jedes Gehirnstatusbildes des Probanden gemäß einem anatomischen Standard-Gehirn;
Mittel zum Ablehnen der Statuswerte von dem kleinsten Wert bis zu dem m-ten kleinsten Wert und Werten von dem größten Wert bis zu dem n-ten größten Wert, von denen angenommen wird, dass sie auf eine Erkrankung hinweisen, in Bezug auf jeden Punkt des Gehirns, wenn die besagten Punkte verwendet werden sollen, um eine Erkrankung basierend auf den besagten anatomisch transformierten und normalisierten Gehirnstatusbildern des Probanden zu diagnostizieren, wobei das Ablehnungsmittel die Gehirnstatuswerte, von denen angenommen wird, dass sie auf eine Erkrankung hinweisen, basierend auf einem Vergleich der Gehirnstatuswerte von Probanden miteinander an jedem Punkt der standardisierten Gehirnbilder identifiziert; und

Mittel zum Erzeugen der Alternative zu einer normalen Gehirndatenbank durch Erhalten mindestens eines mittleren Statuswertes für besagten jeden Punkt des Gehirns, der zu verwenden ist, um eine Erkrankung basierend auf einem entsprechenden Punkt auf den besagten anatomisch transformierten und normalisierten Gehirnstatusbildern jedes Probanden zu diagnostizieren, in denen die Statuswerte, von denen angenommen wird, dass sie auf eine Erkrankung hinweisen, abgelehnt werden, wobei ein Mittelwertberechnungsmittel den Mittelwert der verbleibenden Gehirnstatuswerte berechnet.

2. Vorrichtung zum Erzeugen einer Alternative zu einer normalen Gehirndatenbank gemäß Anspruch 1, wobei das besagte Ablehnungsmittel die extrem abweichenden Gehirnstatuswerte, die statistisch an jedem Punkt erhalten werden, ablehnt, wenn von den besagten extrem abweichenden Gehirnstatuswerten angenommen wird, dass sie auf eine Erkrankung hinweisen.

3. Vorrichtung zum Erzeugen einer Alternative zu einer normalen Gehirndatenbank gemäß Anspruch 1 oder 2, wobei das besagte Gehirnstatusbild ein Gehirnblutstrombild ist, das den mit einem Blutstrom assoziierten Wert jedes Punktes des Gehirns anzeigt.

4. Vorrichtung zum Erzeugen einer Alternative zu einer normalen Gehirndatenbank gemäß Anspruch 1, wobei die Zahl m größer als die Zahl n ist.

5. Vorrichtung zum Erzeugen einer Alternative zu einer normalen Gehirndatenbank gemäß einem der Ansprüche 1 bis 4, wobei

das besagte Erzeugungsmittel die Alternative zu einer normalen Gehirndatenbank durch Erhalten eines mittleren Statuswertes und einer Standardabweichung für besagten jeden Punkt des Gehirns, der zu verwenden ist, um eine Erkrankung basierend auf den anatomisch transformierten und normalisierten Gehirnstatusbildern von Probanden zu diagnostizieren, in denen die Statuswerte, von denen angenommen wird, dass sie auf eine Erkrankung hinweisen, abgelehnt werden, erzeugt.

6. Vorrichtung zum Erzeugen einer Alternative zu einer normalen Gehirndatenbank gemäß einem der Ansprüche 1 bis 5, die ferner aufweist:

Mittel zum Erzeugen eines Gehirnoberflächenstatusbildes jedes Probanden basierend auf den besagten anatomisch transformierten und normalisierten Gehirnstatusbildern der Probanden, wobei das besagte Gehirnoberflächenstatusbild einen Oberflächenstatuswert jedes Oberflächenabschnitts hat, wobei der besagte Oberflächenstatuswert als ein repräsentativer Wert der Statuswerte der Gehirnoberfläche bis zu einer vorbestimmten Tiefe senkrecht zu der Gehirnoberfläche ausgewählt wird; und wobei besagter jeder Oberflächenpunkt, an dem der Oberflächenstatuswert angezeigt wird, als der besagte Punkt des Gehirns, der zu verwenden ist, um eine Erkrankung zu diagnostizieren, verwendet wird.

7. Computerprogramm zum Erzeugen einer Alternative zu einer normalen Gehirndatenbank, die zu verwenden ist, um eine Gehirnerkrankung basierend auf den Gehirnstatusbildern jedes Probanden aus einer Reihe von Probanden, die Patienten umfassen, zu diagnostizieren, wobei die Alternative zu einer normalen Gehirndatenbank einen normalen Status von jedem Punkt des Gehirns anzeigt, wobei das Programm Anweisungen für die Schritte aufweist:

Normalisieren des Statuswertes an jedem Punkt jedes Gehirnstatusbildes eines Probanden basierend auf den Statuswerten eines Teils oder Ganzen des Gehirnstatusbildes des Probanden; räumliches Transformieren jedes Gehirnstatusbildes des Probanden gemäß einem anatomischen Standard-Gehirn; Ablehnen der Statuswerte von dem kleinsten Wert bis zu dem m-ten kleinsten Wert und Werten von dem größten Wert bis zu dem n-ten größten Wert, von denen angenommen wird, dass sie auf eine Erkrankung hinweisen, in Bezug auf jeden Punkt des Gehirns, wenn die besagten Punkte verwendet werden sollen, um eine Erkrankung basierend auf den anatomisch transformierten und normalisierten Gehirnstatusbildern des Probanden zu diagnostizieren, wobei das Ablehnungsmittel die Gehirnstatuswerte, von denen angenommen wird, dass sie auf eine Erkrankung hinweisen, basierend auf einem Vergleich der Gehirnstatuswerte von Probanden miteinander, an jedem Punkt der standardisierten Gehirnbilder identifiziert; und

Erzeugen der Alternative zu einer normalen Gehirndatenbank durch Erhalten mindestens eines mittleren Statuswertes für besagten jeden Punkt des Gehirns, der zu verwenden ist, um eine Erkrankung basierend auf einem entsprechenden Punkt auf den anatomisch transformierten und normalisierten Gehirnstatusbildern jedes Probanden zu diagnostizieren, in denen die Statuswerte, von denen angenommen wird, dass sie auf eine Erkrankung hinweisen, abgelehnt werden, wobei ein Mittelwertberechnungsmittel den Mittelwert der verbleibenden Gehirnstatuswerte berechnet.

8. Computerprogramm zur Erzeugung einer Alternative zu einer normalen Gehirndatenbank gemäß Anspruch 7, wobei die besagten abgelehnten Statuswerte in dem Ablehnungsschritt extrem abweichende Werte, die statistisch an jedem Punkt als die besagten Statuswerte erhalten werden, sind, von denen angenommen wird, dass sie auf eine Erkrankung hinweisen.

9. Computerprogramm zur Erzeugung einer Alternative zu einer normalen Gehirndatenbank gemäß Anspruch 7 oder 8, wobei
   das besagte Gehirnstatusbild ein Gehirnblutstrombild ist, das einen mit einem Blutstrom assoziierten Wert jedes Punktes des Gehirns anzeigt.

10. Computerprogramm zur Erzeugung einer Alternative zu einer normalen Gehirndatenbank gemäß Anspruch 9, wobei die Zahl m größer als die Zahl n ist.

11. Computerprogramm zur Erzeugung einer Alternative zu einer normalen Gehirndatenbank gemäß einem der Ansprüche 7 bis 10, wobei
    in dem besagten Erzeugungsschritt die Alternative zu einer normalen Gehirndatenbank durch Erhalten eines mittleren Statuswertes und einer Standardabweichung für besagten jeden Punkt des Gehirns, der zu verwenden ist, um eine Erkrankung basierend auf den anatomisch transformierten und normalisierten Gehirnstatusbildern von den Probanden zu diagnostizieren, in denen die besagten Statuswerte angenommen werden, um auf eine Erkrankung hinzuweisen, abgelehnt werden, erzeugt wird.

12. Computerprogramm zur Erzeugung einer Alternative zu einer normalen Gehirndatenbank gemäß einem der Ansprüche 7 bis 10, das ferner Anweisungen für den Schritt aufweist:

    Erzeugen eines Gehirnoberflächenstatusbildes jedes Probanden basierend auf den besagten anatomisch transformierten und normalisierten Gehirnstatusbildern der Probanden, wobei das besagte Gehirnoberflächenstatusbild einen Oberflächenstatuswert jedes Oberflächenabschnitts hat, wobei der besagte Oberflächenstatuswert als ein repräsentativer Wert der Statuswerte der Gehirnoberfläche bis zu einer vorbestimmten Tiefe senkrecht zu der Gehirnoberfläche ausgewählt wird; und
    wobei besagter jeder Oberflächenpunkt, an dem der Oberflächenstatuswert angezeigt wird, als der besagte Punkt des Gehirns, der zu verwenden ist, um eine Erkrankung zu diagnostizieren, verwendet wird.

13. Verfahren zum Erzeugen einer Alternative zu einer normalen Gehirndatenbank, die zu verwenden ist, um eine Gehirnerkrankung basierend auf Gehirnstatusbildern jedes Probanden aus einer Reihe von Probanden, die Patienten umfassen, zu diagnostizieren, wobei die Alternative zu einer normalen Gehirndatenbank einen normalen Status von jedem Punkt des Gehirns anzeigt, wobei das Verfahren die Schritte aufweist:

    Normalisieren des Statuswertes an jedem Punkt jedes Gehirnstatusbildes eines Probanden basierend auf den Statuswerten eines Teils oder Ganzen des Gehirnstatusbildes des Probanden;
    räumliches Transformieren jedes Gehirnstatusbildes des Probanden gemäß einem anatomischen Standard-Gehirn;
    Ablehnen der Statuswerte von dem kleinsten Wert bis zu dem m-ten kleinsten Wert und Werten von dem größten Wert bis zu dem n-ten größten Wert, von denen angenommen wird, dass sie auf eine Erkrankung hinweisen, in Bezug auf jeden Punkt des Gehirns, wenn die besagten Punkte verwendet werden sollen, um eine Erkrankung basierend auf den anatomisch transformierten und normalisierten Gehirnstatusbildern des Probanden zu diagnostizieren, wobei das Ablehnungsmittel die Gehirnstatuswerte, von denen angenommen wird, dass sie auf eine Erkrankung hinweisen, basierend auf einem Vergleich der Gehirnstatuswerte von Probanden miteinander an jedem Punkt der standardisierten Gehirnbilder identifiziert;
    und
    Erzeugen der Alternative zu einer normalen Gehirndatenbank durch Erhalten mindestens eines mittleren Statuswertes für besagten jeden Punkt des Gehirns, der zu verwenden ist, um eine Erkrankung basierend auf einem entsprechenden Punkt auf den anatomisch transformierten und normalisierten Gehirnstatusbildern jedes Probanden zu diagnostizieren, in denen die Statuswerte, von denen angenommen wird, dass sie auf eine Erkrankung hinweisen, abgelehnt werden, wobei ein Mittelwertberechnungsmittel den Mittelwert der verbleibenden Gehirnstatuswerte berechnet.

14. Verfahren zur Erzeugung einer Alternative zu einer normalen Gehirndatenbank gemäß Anspruch 13, wobei die besagten abgelehnten Statuswerte in dem Ablehnungsschritt extrem abweichende Werte, die statistisch an jedem Punkt als die besagten Statuswerte erhalten werden, sind, von denen angenommen wird, dass sie auf eine Erkrankung hinweisen.

15. Verfahren zur Erzeugung einer Alternative zu einer normalen Gehirndatenbank gemäß Anspruch 13 oder 14, wobei das besagte Gehirnstatusbild ein Gehirnblutstrombild ist, das einen mit einem Blutstrom assoziierten Wert jedes Punktes des Gehirns anzeigt.

16. Verfahren zur Erzeugung einer Alternative zu einer normalen Gehirndatenbank gemäß Anspruch 13, wobei die Zahl m größer als die Zahl n ist.

17. Verfahren zur Erzeugung einer Alternative zu einer normalen Gehirndatenbank gemäß einem der Ansprüche 13 bis 16, wobei
in dem besagten Erzeugungsschritt die Alternative zu einer normalen Gehirndatenbank durch Erhalten eines mittleren Statuswertes und einer Standardabweichung für besagten jeden Punkt des Gehirns, der zu verwenden ist, um eine Erkrankung basierend auf den anatomisch transformierten und normalisierten Gehirnstatusbildern von den Probanden zu diagnostizieren, in denen die besagten Statuswerte angenommen werden, um auf eine Erkrankung hinzuweisen, abgelehnt werden, erzeugt wird.

18. Verfahren zur Erzeugung einer Alternative zu einer normalen Gehirndatenbank gemäß einem der Ansprüche 13 bis 17, das ferner den Schritt aufweist:

Erzeugen eines Gehirnoberflächenstatusbildes jedes Probanden basierend auf den besagten anatomisch transformierten und normalisierten Gehirnstatusbildern der Probanden, wobei das besagte Gehirnoberflächenstatusbild einen Oberflächenstatuswert jedes Oberflächenabschnitts hat, wobei der besagte Oberflächenstatuswert als ein repräsentativer Wert der Statuswerte der Gehirnoberfläche bis zu einer vorbestimmten Tiefe senkrecht zu der Gehirnoberfläche ausgewählt wird; und
wobei besagter jeder Oberflächenpunkt, an dem der Oberflächenstatuswert angezeigt wird, als der besagte Punkt des Gehirns, der zu verwenden ist, um eine Erkrankung zu diagnostizieren, verwendet wird.

## Revendications

1. Dispositif de génération d'une base de données alternative pour cerveau normal à utiliser pour diagnostiquer une maladie du cerveau sur la base d'une pluralité d'images d'état du cerveau de chaque sujet d'un ensemble de sujets incluant des patients, la base de données alternative pour cerveau normal montrant un état normal de chaque point d'un cerveau, le dispositif comprenant :

un moyen de normalisation d'une valeur d'état à chaque point de chaque image d'état du cerveau d'un sujet sur la base des valeurs d'état d'une partie ou de l'ensemble de l'image d'état du cerveau du sujet ;
un moyen de transformation spatiale de chaque image d'état du cerveau du sujet en fonction d'un cerveau standard anatomique ;
un moyen de rejet des valeurs d'état de la valeur la plus petite à la m-ième valeur la plus petite et des valeurs de la valeur la plus grande à la n-ième valeur la plus grande supposée indiquer une maladie par rapport à chaque point du cerveau, lorsque lesdits points sont à utiliser pour diagnostiquer une maladie sur la base desdites images d'état du sujet anatomiquement transformées et normalisées, dans lequel le moyen de rejet identifie les valeurs d'état du cerveau supposées indiquer une maladie sur la base d'une comparaison des valeurs d'état du cerveau de sujets les unes avec les autres à chaque point des images du cerveau standardisées ;
et
un moyen de génération de la base de données alternative pour cerveau normal par obtention d'au moins une valeur d'état moyenne pour ledit chaque point du cerveau à utiliser pour diagnostiquer une maladie sur la base d'un point respectif sur chacune desdites images d'état du cerveau anatomiquement transformées et normalisées du sujet dans lesquelles lesdites valeurs d'état supposées indiquer une maladie sont rejetées, dans lequel un moyen de calcul de valeur moyenne calcule la valeur moyenne des valeurs d'état du cerveau restantes.

2. Dispositif de génération d'une base de données alternative pour cerveau normal selon la revendication 1, dans lequel ledit moyen de rejet rejette les valeurs d'état du cerveau d'écart extrême, obtenues statistiquement à chaque point, lorsque lesdites valeurs d'état du cerveau d'écart extrême sont supposées indiquer une maladie.

3. Dispositif de génération d'une base de données alternative pour cerveau normal selon les revendications 1 ou 2, dans lequel
ladite image d'état du cerveau est une image de circulation sanguine du cerveau qui montre la valeur associée à la circulation sanguine de chaque point du cerveau.

4. Dispositif de génération d'une base de données alternative pour cerveau normal selon la revendication 1, dans lequel le nombre m est supérieur au nombre n.

5. Dispositif de génération d'une base de données alternative pour cerveau normal selon l'une quelconque des revendications 1 à 4, dans lequel
ledit moyen de génération génère la base de données alternative pour cerveau normal par obtention d'une valeur d'état moyenne et d'un écart type pour ledit chaque point du cerveau, à utiliser pour diagnostiquer une maladie sur la base desdites images d'état du cerveau anatomiquement transformées et normalisées de sujets dans lesquelles lesdites valeurs d'état supposées indiquer une maladie sont rejetées.

6. Dispositif de génération d'une base de données alternative pour cerveau normal selon l'une quelconque des revendications 1 à 5, comprenant en outre :

un moyen de génération d'une image d'état de surface du cerveau de chaque sujet sur la base desdites images d'état du cerveau anatomiquement transformées et normalisées des sujets, ladite image d'état de surface du cerveau ayant une valeur d'état de surface de chaque portion de surface, ladite valeur d'état de surface étant sélectionnée en tant que valeur représentative des valeurs d'état de la surface du cerveau à une profondeur prédéterminée perpendiculaire à la surface du cerveau ; et
dans lequel ledit chaque point de surface auquel ladite valeur d'état de surface est indiquée est utilisé comme ledit point du cerveau à utiliser pour diagnostiquer une maladie.

7. Programme informatique de génération d'une base de données alternative pour cerveau normal à utiliser pour diagnostiquer une maladie du cerveau sur la base des images d'état du cerveau de chaque sujet d'un ensemble de sujets incluant des patients, la base de données alternative pour cerveau normal montrant un état normal de chaque point du cerveau, le programme comprenant des instructions pour les étapes de :

normalisation de la valeur d'état à chaque point de chaque image d'état du cerveau d'un sujet sur la base des valeurs d'état d'une partie ou de l'ensemble de l'image d'état du cerveau du sujet ;
transformation spatiale de chaque image d'état du cerveau du sujet en fonction d'un cerveau standard anatomique ;
rejet des valeurs d'état de la valeur la plus petite à la m-ième valeur la plus petite et des valeurs de la valeur la plus grande à la n-ième valeur la plus grande supposée indiquer une maladie par rapport à chaque point du cerveau, lorsque lesdits points sont à utiliser pour diagnostiquer une maladie sur la base desdites images d'état du sujet anatomiquement transformées et normalisées, dans lequel le moyen de rejet identifie les valeurs d'état du cerveau supposées indiquer une maladie sur la base d'une comparaison des valeurs d'état du cerveau de sujets les unes avec les autres à chaque point des images du cerveau standardisées ;
et
génération de la base de données alternative pour cerveau normal par obtention d'au moins une valeur d'état moyenne pour ledit chaque point du cerveau à utiliser pour diagnostiquer une maladie sur la base d'un point respectif sur chacune desdites images d'état du cerveau anatomiquement transformées et normalisées du sujet dans lesquelles lesdites valeurs d'état supposées indiquer une maladie sont rejetées, dans lequel un moyen de calcul de valeur moyenne calcule la valeur moyenne des valeurs d'état du cerveau restantes.

8. Programme informatique de génération d'une base de données alternative pour cerveau normal selon la revendication 7, dans lequel

lesdites valeurs d'état rejetées dans ladite étape de rejet sont des valeurs d'écart extrême obtenues statistiquement à chaque point lorsque lesdites valeurs d'état sont supposées indiquer une maladie.

9. Programme informatique de génération d'une base de données alternative pour cerveau normal selon les revendications 7 ou 8, dans lequel
ladite image d'état du cerveau est une image de circulation sanguine du cerveau qui montre une valeur associée à la circulation sanguine de chaque point du cerveau.

10. Programme informatique de génération d'une base de données alternative pour cerveau normal selon la revendication 9, dans lequel
le nombre m est supérieur au nombre n.

11. Programme informatique de génération d'une base de données alternative pour cerveau normal selon l'une quelconque des revendications 7 à 10, dans lequel
dans ladite étape de génération, la base de données alternative pour cerveau normal est générée par obtention d'une valeur d'état moyenne et d'un écart type pour ledit chaque point du cerveau à utiliser pour diagnostiquer une maladie sur la base desdites images d'état du cerveau anatomiquement transformées et normalisées des sujets dans lesquelles lesdites valeurs d'état sont supposées indiquer une maladie sont rejetées.

12. Programme informatique de génération d'une base de données alternative pour cerveau normal selon l'une quelconque des revendications 7 à 11, comprenant en outre une instruction pour l'étape de :

génération d'une image d'état de surface du cerveau de chaque sujet sur la base desdites images d'état du cerveau anatomiquement transformées et normalisées des sujets, ladite image d'état de surface du cerveau ayant une valeur d'état de surface de chaque portion de surface, ladite valeur d'état de surface étant sélectionnée en tant que valeur représentative des valeurs d'état de la surface du cerveau à une profondeur prédéterminée perpendiculaire à la surface du cerveau ; et
dans lequel ledit chaque point de surface auquel ladite valeur d'état de surface est indiquée est utilisé comme ledit point du cerveau à utiliser pour diagnostiquer une maladie.

13. Procédé de génération d'une base de données alternative pour cerveau normal à utiliser pour diagnostiquer une maladie du cerveau sur la base d'images d'état du cerveau de chaque sujet d'un ensemble de sujets incluant des patients, la base de données alternative pour cerveau normal montrant un état normal de chaque point du cerveau, le procédé comprenant les étapes de :

normalisation d'une valeur d'état à chaque point de chaque image d'état du cerveau d'un sujet sur la base des valeurs d'un état d'une partie ou de l'ensemble de l'image d'état du cerveau du sujet ;
transformation spatiale de chaque image d'état du cerveau du sujet en fonction d'un cerveau standard anatomique ;
rejet des valeurs d'état de la valeur la plus petite à la m-ième valeur la plus petite et des valeurs de la valeur la plus grande à la n-ième valeur la plus grande supposée indiquer une maladie par rapport à chaque point du cerveau, lorsque lesdits points sont à utiliser pour diagnostiquer une maladie sur la base desdites images d'état du sujet anatomiquement transformées et normalisées, dans lequel le moyen de rejet identifie les valeurs d'état du cerveau supposées indiquer une maladie sur la base d'une comparaison des valeurs d'état du cerveau de sujets les unes avec les autres à chaque point des images du cerveau standardisées ;
et
génération de la base de données alternative pour cerveau normal par obtention d'au moins une valeur d'état moyenne pour ledit chaque point du cerveau à utiliser pour diagnostiquer une maladie sur la base d'un point respectif sur chacune desdites images d'état du cerveau anatomiquement transformées et normalisées du sujet dans lesquelles lesdites valeurs d'état supposées indiquer une maladie sont rejetées, dans lequel un moyen de calcul de valeur moyenne calcule la valeur moyenne des valeurs d'état du cerveau restantes.

14. Procédé de génération d'une base de données alternative pour cerveau normal selon la revendication 13, dans lequel lesdites valeurs d'état rejetées dans ladite étape de rejet sont des valeurs d'écart extrême obtenues statistiquement à chaque point lorsque lesdites valeurs d'état sont supposées indiquer une maladie.

15. Procédé de génération d'une base de données alternative pour cerveau normal selon les revendications 13 ou 14, dans lequel

ladite image d'état du cerveau est une image de circulation sanguine du cerveau qui montre une valeur associée à la circulation sanguine de chaque point du cerveau.

16. Procédé de génération d'une base de données alternative pour cerveau normal selon la revendication 13, dans lequel le nombre m est supérieur au nombre n.

17. Procédé de génération d'une base de données alternative pour cerveau normal selon l'une quelconque des revendications 13 à 16, dans lequel
dans ladite étape de génération, la base de données alternative pour cerveau normal est générée par obtention d'une valeur d'état moyenne et d'un écart type pour ledit chaque point du cerveau à utiliser pour diagnostiquer une maladie sur la base desdites images d'état du cerveau anatomiquement transformées et normalisées des sujets dans lesquelles lesdites valeurs d'état sont supposées indiquer une maladie sont rejetées.

18. Procédé de génération d'une base de données alternative pour cerveau normal selon l'une quelconque des revendications 13 à 17, comprenant en outre l'étape de :

génération d'une image d'état de surface du cerveau de chaque sujet sur la base desdites images d'état du cerveau anatomiquement transformées et normalisées des sujets, ladite image d'état de surface du cerveau ayant une valeur d'état de surface de chaque portion de surface, ladite valeur d'état de surface étant sélectionnée en tant que valeur représentative de valeurs d'état de la surface du cerveau à une profondeur prédéterminée perpendiculaire à la surface du cerveau ; et
dans lequel ledit chaque point de surface auquel ladite valeur d'état de surface est indiquée est utilisé comme ledit point du cerveau à utiliser pour diagnostiquer une maladie.

[Fig. 1]

FIG.1

[Fig. 2]

FIG.2

# FIG.3

[Fig. 3]

EP 2 346 407 B1

[Fig. 4]

# FIG.4

EP 2 346 407 B1

[Fig. 5]

# FIG.5

EP 2 346 407 B1

[Fig. 6]

# FIG.6

PROGRAM FOR GENERATING ALTERNATIVE
NORMAL BRAIN DATABASE

```
        ( START )
            |
            v
  OBTAINING BRAIN BLOODSTREAM    ─ S1
   ASSOCIATED VALUES AND
       NORMALIZING
            |
            v
  ANATOMICAL STANDARDIZATION     ─ S2
            |
            v
   GENERATING ALTERNATIVE        ─ S3
   NORMAL BRAIN DATABASE
            |
            v
        ( END )
```

[Fig. 7]

# FIG.7

OBTAINING BRAIN BLOODSTREAM ASSOCIATED
VALUES AND NORMALIZING

START

OBTAINING SPECT DATA — S11

RECONSTRUCTING 3-D IMAGE
DATA OF BRAIN BLOODSTREAM — S12

NORMALIZATION — S13

END

[Fig. 8]

# FIG.8

| X, Y, Z | NORMALIZED BLOODSTREAM ASSOCIATED VALUE |
|---|---|
| 0, 0, 0 | 0.00 |
| 0, 0, 1 | 0.00 |
| 0, 0, 2 | 0.00 |
| ⋮ | ⋮ |
| 115, 98, 102 | 1.55 |
| 115, 98, 103 | 1.52 |
| 115, 98, 104 | 1.54 |
| ⋮ | ⋮ |

[Fig. 9]

# FIG.9

[Fig. 10]

# FIG.10

## ANATOMICAL STANDARDIZATION

```
                    ( START )
                        │
                        ▼
┌──────────────────────────────────────────────┐  S21
│        READING OUT NORMALIZED 3-D             │
│     BLOODSTREAM IMAGE OF INITIAL SUBJECT       │
└──────────────────────────────────────────────┘
  ( 2 )─────────────────────►│
                        ▼
┌──────────────────────────────────────────────┐  S22
│        IDENTIFYING INTERHEMISPHERIC            │
│            MID-SAGITTAL PLANE                  │
└──────────────────────────────────────────────┘
                        │
                        ▼
┌──────────────────────────────────────────────┐  S23
│         IDENTIFYING AC-PC LINE AND            │
│             CENTER THEREOF                     │
└──────────────────────────────────────────────┘
                        │
                        ▼
┌──────────────────────────────────────────────┐  S24
│   POSITIONING AC-PC LINE AND CENTER OF        │
│     SUBJECT'S IMAGE TO THAT OF STANDARD        │
│             BRAIN IMAGE                         │
└──────────────────────────────────────────────┘
                        │
                        ▼
┌──────────────────────────────────────────────┐  S25
│    LINEARLY TRANSFORMING SUBJECT'S            │
│   BLOODSTREAM IMAGE IN ACCORDANCE WITH         │
│            STANDARD BRAIN IMAGE                 │
└──────────────────────────────────────────────┘
                        │
                        ▼
┌──────────────────────────────────────────────┐  S26
│   NON-LINEARLY TRANSFORMING SUBJECT'S         │
│     BLOODSTREAM IMAGE SO THAT PROFILE          │
│  CURVE OF THE SUBJECT'S BLOODSTREAM IMAGE      │
│ ACCORDS TO THAT OF THE STANDARD BRAIN IMAGE    │
└──────────────────────────────────────────────┘
                        │
                        ▼
                     ( 1 )
```

[Fig. 11]

# FIG.11

S27 — GENERATING BRAIN SURFACE BLOODSTREAM IMAGE

S28 — STORING BRAIN BLOODSTREAM IMAGES AND BRAIN SURFACE BLOODSTREAM IMAGES

S29 — HAVE ALL SUBJECT'S BLOODSTREAM IMAGES BEEN PROCESSED?

NO → S30 — READING OUT NEXT SUBJECT'S BLOODSTREAM IMAGE → 2

YES → END

[Fig. 12]

# FIG.12

### IDENTIFYING THE INTERHEMISPHERIC MID-SAGITTAL PLANE

```
          ( START )
             │
             ▼
┌───────────────────────────────────┐  S221
│        LOOP1    dX= -I,I          │
└───────────────────────────────────┘
             │
             ▼
┌───────────────────────────────────┐  S222
│  ORIGINAL POINT OF COORDINATE IS  │
│  SET AT CENTER POINT OF THE       │
│  BLOODSTREAM IMAGE                │
└───────────────────────────────────┘
             │
             ▼
┌───────────────────────────────────┐  S223
│      LOOP2    d r z= -r I, r I    │
└───────────────────────────────────┘
             │
             ▼
┌───────────────────────────────────┐  S224
│      LOOP3    d r Y= -r I, r I    │
└───────────────────────────────────┘
             │
             ▼
┌───────────────────────────────────┐  S225
│ ROTATING Y-Z PLANE WITH d r z AND │
│ d r Y AROUND Z AXIS AND Y AXIS    │
│ AND ROTATED Y-Z PLANE IS ASSUMED  │
│ AS INTERHEMISPHERIC MID-SAGITTAL  │
│ PLANE                             │
└───────────────────────────────────┘
             │
             ▼
┌───────────────────────────────────┐  S226
│ GENERATING FLIPPED 3-D            │
│ BLOODSTREAM IMAGE BY FLIPPING     │
│ RIGHT SIDE OF 3-D BLOODSTREAM     │
│ IMAGE                             │
└───────────────────────────────────┘
             │
             ▼
┌───────────────────────────────────┐  S227
│ CALCULATING AND STORING           │
│ SIMILARITY INDEX BETWEEN ORIGINAL │
│ AND FLIPPED 3-D BLOODSTREAM IMAGE │
└───────────────────────────────────┘
             │
             ▼
┌───────────────────────────────────┐  S228
│     LOOP1, LOOP2, LOOP3 END       │
└───────────────────────────────────┘
             │
             ▼
           ( 3 )
```

[Fig. 13]

# FIG.13

```
        ( 3 )
          │
          ▼
┌──────────────────────────────────────┐  S229
│    IDENTIFYING INTERHEMISPHERIC        │
│ MID-SAGITTAL PLANE HAVING THE LARGEST  │
│  SIMILARITY INDEX AMONG THE ASSUMED    │
│  INTERHEMISPHERIC MID-SAGITTAL PLANE   │
│      DECIDED BY dX, drz AND drʏ        │
└──────────────────────────────────────┘
          │
          ▼
       ( END )
```

[Fig. 14]

# FIG.14

[Fig. 15]

# FIG.15

## IDENTIFYING AC-PC LINE AND CENTER THEREOF

( START )

READING OUT SECTIONAL BLOODSTREAM IMAGES
AT THE INTERHEMISPHERIC MID-SAGITTAL PLANE
AND PLANES PARALLEL THERETO — S231

DETECTING OUTER BORDER AND
BORDER BETWEEN WHITE AND GRAY MATTERS — S232

DECIDING OCCIPITAL PALE POINT (OP)
AS THE MOST POSTERIOR POINT OF THE
BORDER IMAGE — S233

DECIDING INFERIOR ASPECT OF THE ANTERIOR
CORPS CALLOSUM (CC) — S234

DECIDING FRONTAL PALE POINT(FP)
AS THE MOST ANTERIOR POINT
OF THE BORDER IMAGE — S235

DECIDING SUBTHALAMIC POINT (TH) — S236

GENERATING AC-PC LINE AS REGRESSION
LINE CONNECTING THROUGH OP, TH,CC AND FP — S237

DECIDING CENTER OF AC-PC LINE — S238

( END )

[Fig. 16]

# FIG.16A

# FIG.16B

# FIG.16C

# FIG.16D

[Fig. 17]

# FIG.17A

# FIG.17B

[Fig. 18]

# FIG.18

[Fig. 19]

# FIG.19A

# FIG.19B

[Fig. 20]

# FIG.20

## GENERATING BRAIN SURFACE BLOODSTREAM IMAGE

```
            ( START )
                |
                v
ASSIGNING ID ON EACH BRAIN SURFACE VOXEL          S271
                |
                v
LOOP1   ID=0,N_ID                                 S272
                |
                v
OBTAINING 6 BLOODSTREAM ASSOCIATED VALUES         S273
FROM BRAIN SURFACE TO 6 VOXEL DEPTH
PERPENDICULAR TO BRAIN SURFACE
                |
                v
ASSIGNING BLOODSTREAM ASSOCIATED VALUE            S274
OF BRAIN SURFACE AS MAXIMUM BLOODSTREAM
ASSOCIATED VALUE AMONG THE 6 BLOODSTREAM
ASSOCIATED VALUE
                |
                v
LOOP1  END                                        S275
                |
                v
             ( END )
```

[Fig. 21]

# FIG.21

## GENERATING ALTERNATIVE NORMAL BRAIN DATABASE

```
                    ( START )
                        │
                        ▼
┌───────────────────────────────────────────┐  ⟋ S31
│              LOOP1  X=0,Nx                  │
└───────────────────────────────────────────┘
                        │
                        ▼
┌───────────────────────────────────────────┐  ⟋ S32
│              LOOP2  Y=0,Ny                  │
└───────────────────────────────────────────┘
                        │
                        ▼
┌───────────────────────────────────────────┐  ⟋ S33
│              LOOP3  Z=0,Nz                  │
└───────────────────────────────────────────┘
                        │
                        ▼
┌───────────────────────────────────────────┐  ⟋ S34
│   READING OUT BLOODSTREAM ASSOCIATED VALUES │
│          I(x, y, z) OF ALL SUBJECTS         │
└───────────────────────────────────────────┘
                        │
                        ▼
┌───────────────────────────────────────────┐  ⟋ S35
│   SORTING BLOODSTREAM ASSOCIATED VALUES IN  │
│             DESCENDING ORDER                │
└───────────────────────────────────────────┘
                        │
                        ▼
┌───────────────────────────────────────────┐  ⟋ S36
│   REJECTING BLOODSTREAM ASSOCIATED VALUE    │
│       RANKED TOP 10% AND BOTTOM 40%         │
└───────────────────────────────────────────┘
                        │
                        ▼
┌───────────────────────────────────────────┐  ⟋ S37
│  CALCULATING MEAN VALUE AND SD OF REMAINING │
│        BLOODSTREAM ASSOCIATED VALUES        │
└───────────────────────────────────────────┘
                        │
                        ▼
┌───────────────────────────────────────────┐  ⟋ S38
│              LOOP 1,2,3 END                 │
└───────────────────────────────────────────┘
                        │
                        ▼
                      ( 4 )
```

[Fig. 22]

# FIG.22

```
        ( 4 )
          |
          v
┌─────────────────────────────────────┐  S39
│        LOOP4   ID=0,NID              │
└─────────────────────────────────────┘
          |
          v
┌─────────────────────────────────────┐  S40
│ READING OUT BLOODSTREAM ASSOCIATED   │
│ VALUES I(ID) OF BRAIN SURFACE        │
│ BLOODSTREAM IMAGE OF ALL SUBJECTS    │
└─────────────────────────────────────┘
          |
          v
┌─────────────────────────────────────┐  S41
│ SORTING BLOODSTREAM ASSOCIATED       │
│ VALUES IN DESCENDING ORDER           │
└─────────────────────────────────────┘
          |
          v
┌─────────────────────────────────────┐  S42
│ REJECTING BLOODSTREAM ASSOCIATED     │
│ VALUE RANKED TOP 10% AND BOTTOM 40%  │
└─────────────────────────────────────┘
          |
          v
┌─────────────────────────────────────┐  S43
│ CALCULATING MEAN VALUE AND SD OF     │
│ REMAINING BLOODSTREAM ASSOCIATED     │
│ VALUES                               │
└─────────────────────────────────────┘
          |
          v
┌─────────────────────────────────────┐  S44
│            LOOP4  END                │
└─────────────────────────────────────┘
          |
          v
       ( END )
```

[Fig. 23]

# FIG.23

**VOXEL (11,25,135)**

| SUBJECT ID | BLOODSTREAM ASSOCIATED VALUE |
|:---:|:---:|
| 5 | 2.03 |
| 9 | 1.51 |
| 3 | 1.50 |
| 15 | 1.47 |
| ⋮ | ⋮ |
| 4 | 0.22 |
| 10 | 0.20 |
| 2 | 0.13 |

[Fig. 24]

# FIG.24

| VOXEL | MEAN VALUE | SD |
|---|---|---|
| 0, 0, 0 | 0 | 0 |
| ⋮ | ⋮ | ⋮ |
| 11, 25, 134 | 1.07 | 0.19 |
| 11, 25, 135 | 1.08 | 0.21 |
| ⋮ | ⋮ | ⋮ |

[Fig. 25]

# FIG.25

| ID | VOXEL | MEAN VALUE | SD |
|---|---|---|---|
| 1 | 10, 18, 125 | 1.21 | 0.15 |
| 2 | 10, 18, 126 | 1.23 | 0.14 |

[Fig. 26]

# FIG.26

## REJECTING PROCESS

START

LOOP1    P=O,M — S351

SETTING BLOODSTREAM ASSOCIATED VALUE
Ip(x,y,z) AS TARGET — S352

CALCULATING MEAN VALUE AND SD OF
BLOODSTREAM ASSOCIATED VALUES I(x,y,z)
OF ALL SUBJECTS — S353

CALCULATING $T_0 = \dfrac{|\,Ip - Imean\,|}{S}$ — S354

S355

NO ── $T_0 > T_{RR}$

YES

REJECTING Ip — S356

LOOP1 END — S357

END

# FIG.27

[Fig. 27]

EP 2 346 407 B1

**TABLE OF Ta**

| RR \ M | 4 | 5 | 6 | 7 | 8 | 9 | 10 | 11 | 12 | 13 | 14 |
|---|---|---|---|---|---|---|---|---|---|---|---|
| 5% | 1.65 | 1.76 | 1.81 | 1.85 | 1.87 | 1.88 | 1.90 | 1.90 | 1.91 | 1.92 | 1.92 |
| 1% | 1.71 | 1.92 | 2.05 | 2.14 | 2.21 | 2.26 | 2.29 | 2.32 | 2.35 | 2.37 | 2.38 |

| RR \ M | 15 | 16 | 17 | 18 | 19 | 20 | 21 | 22 | 23 | 24 | 25 |
|---|---|---|---|---|---|---|---|---|---|---|---|
| 5% | 1.92 | 1.93 | 1.93 | 1.93 | 1.93 | 1.93 | 1.94 | 1.94 | 1.94 | 1.94 | 1.94 |
| 1% | 2.40 | 2.41 | 2.42 | 2.43 | 2.44 | 2.45 | 245 | 2.46 | 2.47 | 2.47 | 2.47 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2007081699 A1

- WO 2008093057 A1

**Non-patent literature cited in the description**

- **CHEN WP et al.** Effect of sample size for normal brain database on diagnostic performance of brain FDG PET for the detection of Alzheimer's disease using automated image analysis. *Nucl Med Commun.,* March 2008, vol. 29 (3), 270-6
- **MINOSHIMA et al.** An Automated Method for Rotational Correction and Centering of Three-Dimensional Functional Brain Images. *J Nucl Med,* 1992, vol. 33, 1579-1585
- **MINOSHIMA et al.** An Automated Detection of the Intercommissural Line for Stereotactic Localization of Functional Brain Images. *J Nucl Med,* 1993, vol. 34, 322-329

- **MINOSHIMA.** An Anatomic Standardization: Linear Scaling and Nonlinear Warping of Functional Brain Images. *J Nucl Med,* 1994, vol. 35, 1528-1537
- **F. MAES et al.** Multimodality Image Registration by Maximization of Mutual Information. *IEEE Transactions on Medical Imaging, (USA),* 1997, vol. 16 (2), 187-198
- **WILLIAM R. THOMPSON.** *The annals of Mathematical Statistics,* December 1935, vol. 6 (4), 214-219
- **FRANK E. GRUBBS.** *The annals of Mathematical Statistics,* March 1950, vol. 21 (1), 27-58